# EUROPEAN PATENT SPECIFICATION

(11) **EP 3 215 151 B1**
(45) Date of publication and mention of the grant of the patent: **16.09.2020**
(21) Application number: 15857452.5
(22) Date of filing: 03.11.2015
(51) Int. Cl.: A61K 31/415, A61P 11/00, A61P 43/00

(54) **RESTORING PHYSIOLOGY WITH SMALL MOLECULE MIMICS OF MISSING PROTEINS**
WIEDERHERSTELLUNG DER PHYSIOLOGIE MIT KLEINMOLEKÜLIGER MIMETIKA VON FEHLENDEN PROTEINE
RÉTABLISSEMENT DE PHYSIOLOGIE À L'AIDE D'UNE IMITATION DE PETITE MOLÉCULE DE PROTÉINES MANQUANTES

(30) Priority: 04.11.2014 US 201462074878 P
(43) Date of publication of application: 13.09.2017
(73) Proprietor: The Board of Trustees of the University of Illinois, Urbana, IL 61801 (US)
(72) Inventor: BURKE, Martin, D., Champaign, IL 61821 (US); CIOFFI, Alexander, G., Urbana, IL 61801 (US); DIAZ, Katrina, A., Urbana, IL 61801 (US); HOU, Jennifer, Champaign, IL 61820 (US); GRILLO, Anthony, S., Champaign, IL 61821 (US)
(74) Representative: HGF
(86) International application number: PCT/US2015/058806
(87) International publication number: WO 2016/073462

(56) References cited:
- US-A- 5 880 101
- US-B1- 6 323 191
- CIOFFI ALEX: "67 - Functional complementation of a deficient protein with a small molecule restores cell physiology", ABSTRACTS OF PAPERS AMERICAN CHEMICAL SOCIETY , 8 September 2013 (2013-09-08), XP002779809, & 246TH NATIONAL MEETING OF THE AMERICAN-CHEMICAL-SOCIETY (ACS); INDIANAPOLIS, IN, USA; SEPTEMBER 08 -12, 2013 ISSN: 0065-7727 Retrieved from the Internet: URL:http://acselb-529643017.us-west-2.elb. amazonaws.com/chem/246nm/program/view.php? obj_id=208519&terms= [retrieved on 2018-04-05]
- GRILLO ANTHONY S ET AL: "Restoring growth in protein deficient yeast with a small molecule", ABSTRACTS OF PAPERS AMERICAN CHEMICAL SOCIETY, vol. 248, August 2014 (2014-08), pages 574-ORGN, XP009504547, & 248TH NATIONAL MEETING OF THE AMERICAN-CHEMICAL-SOCIETY (ACS); SAN FRANCISCO, CA, USA; AUGUST 10 -14, 2014 ISSN: 0065-7727
- KIM JUNE-BUM: "Channelopathies.", KOREAN JOURNAL OF PEDIATRICS, vol. 57, no. 1, January 2014 (2014-01), pages 1-18, XP002779810, ISSN: 1738-1061
- HENNEQUIN C ET AL: "In vitro susceptibilities to amphotericin B, intraconazole, and miconazole of filamentous fungi isolated from patients with cystic fibrosis", ANTIMICROBIAL AGENTS AND CHEMOTHERAPY, vol. 41, no. 9, 1997, pages 2064-2066, XP002779811, ISSN: 0066-4804
- R. E. LEWIS ET AL: "Comparative Analysis of Amphotericin B Lipid Complex and Liposomal Amphotericin B Kinetics of Lung Accumulation and Fungal Clearance in a Murine Model of Acute Invasive Pulmonary Aspergillosis", ANTIMICROBIAL AGENTS AND CHEMOTHERAPY, vol. 51, no. 4, 1 April 2007 (2007-04-01), pages 1253-1258, XP055076516, ISSN: 0066-4804, DOI: 10.1128/AAC.01449-06
- ANDERSON ET AL.: 'Amphotericin forms an extramembranous and fungicidal sterol sponge' NAT. CHEM. BIOL. vol. 10, no. 5, 01 May 2014, pages 400 - 406, XP055440085
- GRAY ET AL.: 'Amphotericin primarily kills yeast by simply binding ergosterol' PNAS vol. 109, no. 7, 14 February 2012, pages 2234 - 2239, XP055440089
- LIVRAGHI ET AL.: 'Modelling Dysregulated Na+ Absorption in Airway Epithelial Cells with Mucosal Nystatin Treatment' AM. J. RESPIR. CELL . MOL. BIOL. vol. 38, no. 4, 07 November 2007, pages 423 - 434, XP055440092

## Description

### BACKGROUND OF THE INVENTION

Cystic fibrosis is a well-known autosomal recessive genetic disease that affects multiple organ systems, notably pulmonary and gastrointestinal systems. It occurs in 1 in 3,000 live births and is caused by mutations in the *CFTR* gene encoding cystic fibrosis transmembrane conductance regulator (CFTR), a membrane-expressed chloride channel protein in vertebrates. Typically characterized by chronic and potentially fatal respiratory infections, cystic fibrosis currently has only symptomatic treatments, and patients have a median survival of only 33 years.

CFTR is an ABC transporter-class ion channel that conducts chloride ions across epithelial cell membranes. Mutations of the CFTR gene affecting chloride ion channel function lead to dysregulation of epithelial fluid transport in the lung, pancreas and other organs, resulting in cystic fibrosis. Complications include thickened mucus in the lungs with frequent respiratory infections, and pancreatic insufficiency giving rise to malnutrition and diabetes. These conditions lead to chronic disability and reduced life expectancy. In male patients, the progressive obstruction and destruction of the developing vas deferens and epididymis appear to result from abnormal intraluminal secretions, causing congenital absence of the vas deferens and male infertility.

CFTR functions as a cAMP-activated ATP-gated anion channel, increasing the conductance for certain anions (e.g. Cl⁻) to flow down their electrochemical gradient. ATP-driven conformational changes in CFTR open and close a gate to allow transmembrane flow of anions down their electrochemical gradient. This is in contrast to other ABC proteins, in which ATP-driven conformational changes fuel uphill substrate transport across cellular membranes. Essentially, CFTR is an ion channel that evolved as a "broken" ABC transporter that leaks when in open conformation.

The CFTR is found in the epithelial cells of many organs including the lung, liver, pancreas, digestive tract, reproductive tract, and skin. Normally, the protein moves chloride and thiocyanate ions (with a negative charge) out of an epithelial cell to the covering mucus. Positively charged sodium ions follow passively, increasing the total electrolyte concentration in the mucus, resulting in the movement of water out of the cell by osmosis.

### SUMMARY OF THE INVENTION

An aspect of the invention is a method of treating a disease or condition characterized by decreased expression or reduced function of an ion channel, comprising administering to a subject in need thereof a therapeutically effective amount of a pore-forming polyene macrolide or pore-forming derivative thereof, thereby treating the disease or condition characterized by decreased expression or reduced function of the ion channel. The pore-forming polyene macrolide or pore-forming derivative thereof permits transmembrane trafficking of ions otherwise regulated by the ion channel.

In certain embodiments, the disease or condition characterized by decreased expression or reduced function of an ion channel is cystic fibrosis.

In certain embodiments, the polyene macrolide is selected from the group consisting of amphotericin B (AmB), nystatin, natamycin (also known as piramicin), and any combination thereof.

In certain embodiments, the polyene macrolide is amphotericin B (AmB).

In certain embodiments, the polyene macrolide is administered in a dose less than its minimum inhibitory concentration for *Saccharomyces cerevisiae.*

In certain embodiments, the subject is a human.

In certain embodiments, the subject is a human less than 12 years old.

In certain embodiments, the subject is a human at least 12 years old.

### BRIEF DESCRIPTION OF THE DRAWINGS

**Figure 1** depicts the chemical structures of amphotericin B (AmB), the archetypical ion channel-forming small molecule, and C35-deoxy amphotericin B (C35deOAmB), a channel-inactivated derivative of AmB.
**Figure 2A** depicts a simplified schematic of a functional complementation experiment in which AmB ion channels are predicted to restore growth in trk1Δtrk2Δ cells via collaborating with the ion pumps Pma1 (plasma membrane ATPase 1) and V-ATPase (vacuolar H⁺-ATPase).
**Figure 2B** depicts growth, on normal potassium (10 mM) YPAD plates, of wild type *S. cerevisiae* yeast cells (left), potassium ion transporter-deficient trk1Δtrk2Δ yeast cells (center), and trk1Δtrk2Δ yeast cells complemented with a low concentration (0.125 µM) of AmB (right).
**Figure 2C** depicts concentration-dependence of AmB-mediated rescue of cell growth observed upon the addition of a paper disc impregnated with AmB to a plate of trk1Δtrk2Δ cells.
**Figure 2D** is a graph depicting trk1Δtrk2Δ cell growth restoration at the indicated concentrations of AmB ( 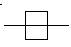 ) and C35deOAmB ( 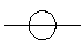 ). Data points represent mean ± SEM.
**Figure 2E** is a graph depicting the effect of tetraethylammonium (TEA), which blocks the AmB ion channel, on AmB-mediated growth restoration observed in trk1Δtrk2Δ cells grown under normal conditions (10 mM KCl, 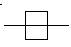 ) and permissive conditions (100 mM KCl, 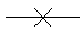 ). Data points represent mean ± SEM.
**Figure 2F** is a graph depicting uptake of extracellular ⁸⁶Rb⁺, a surrogate for K⁺, in wild type *S. cerevisiae* ( 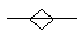 ), untreated trk1Δtrk2Δ cells ( 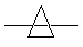 ), AmB-treated trk1Δtrk2Δ cells ( 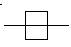 ), and C35deOAmB-treated trk1Δtrk2Δ cells ( 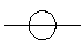 ). Data points represent mean ± SEM.
**Figure 2G** is a bar graph depicting maximum OD₆₀₀ of liquid cultures (after 24 h) of wild type *S. cerevisiae,* untreated trk1Δtrk2Δ cells, and AmB-treated trk1Δtrk2Δ cells. NS, not significant. **** *P* ≤ 0.0001.
**Figure 2H** is a bar graph depicting cell viability, as judged by propidium iodide staining, of wild type *S. cerevisiae* and AmB-treated trk1Δtrk2Δ cells. NS, not significant.
**Figure 2I** is a graph depicting duration of growth of wild type *S. cerevisiae* ( 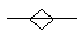 ) and AmB-rescued trk1Δtrk2Δ cells ( 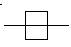 ). Data points represent mean ± SEM.
**Figure 3A** depicts cell growth of trk1Δtrk2Δ yeast treated with any of a number of potassium-transporting polyene macrolide natural products and other small molecules that transport other ions.
**Figure 3B** is a graph depicting sensitivity of AmB-treated wild type *S. cerevisiae* (
   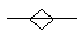 ) and trk1Δtrk2Δ ( 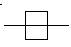 ) cells to the off-pathway microtubule inhibitor nocodazole. Data points represent mean ± SEM.
**Figure 3C** is a graph depicting sensitivity of wild type *S. cerevisiae* ( 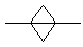 ) and AmB-rescued trk1Δtrk2Δ cells ( 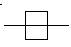 ) to the Pma1 inhibitor ebselen. Data points represent mean ± SEM.
**Figure 3D** is a graph depicting sensitivity of wild type *S. cerevisiae* ( 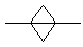 ) and AmB-rescued trk1Δtrk2Δ cells ( 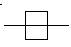 ) to the V-ATPase inhibitor bafilomycin. Data points represent mean ± SEM.
**Figure 3E** is a bar graph depicting EC₅₀ values for nocodazole, an inhibitor of microtuble dynamics, against wild type (black bars) and trk1Δtrk2Δ cells (white bars) treated with optimum rescue concentrations of AmB (0.5 µM), nystatin (1 µM), candicidin (0.008 µM), and mepartricin (0.008 µM). Data points represent mean ± SEM. NS, not significant. * *P* ≤ 0.05.
**Figure 3F** is a bar graph depicting EC₅₀ values for ebselen, a Pma1 inhibitor, against wild type (black bars) and trk1Δtrk2Δ cells (white bars) treated with optimum rescue concentrations of AmB (0.5 µM), nystatin (1 µM), candicidin (0.008 µM), and mepartricin (0.008 µM). *** *P* ≤ 0.001. **** *P* ≤ 0.0001.
**Figure 3G** is a bar graph depicting EC₅₀ values for bafilomycin, a V-ATPase inhibitor, against wild type (black bars) and trk1Δtrk2Δ cells (white bars) treated with optimum rescue concentrations of AmB (0.5 µM), nystatin (1 µM), candicidin (0.008 µM), and mepartricin (0.008 µM). Data points represent mean ± SEM. **** *P* ≤ 0.0001.

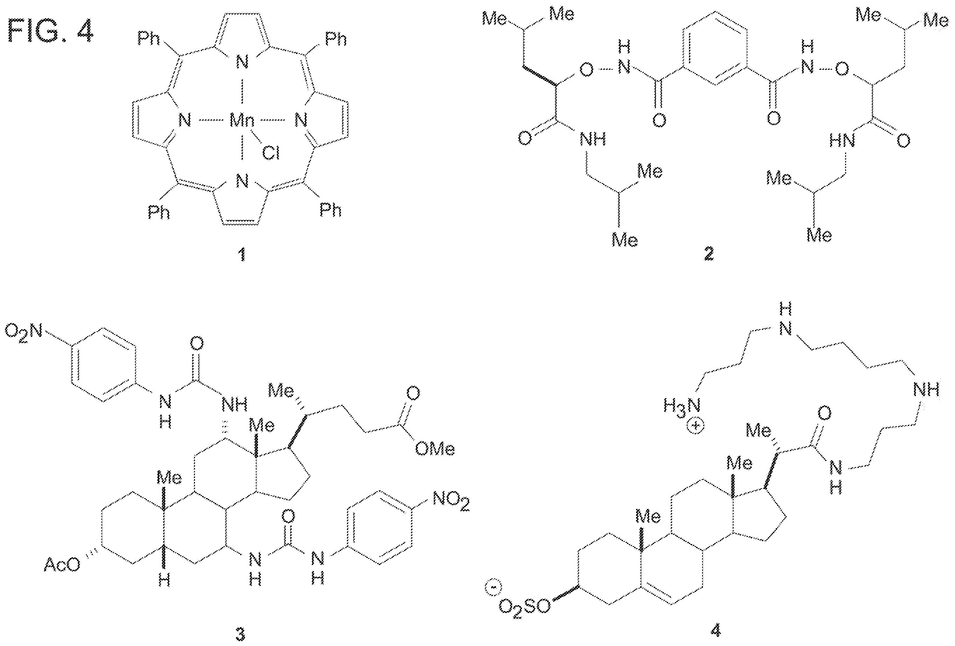

**Figure 4** depicts compounds **1-4** previously reported to permeabilize to chloride planar lipid bilayers, liposomes, and/or cells.
**Figure 5A** is a series of three confocal fluorescence microscopy images depicting airway surface liquid (ASL) height in vehicle-treated normal human lung epithelia (left), vehicle-treated CF human lung epithelia (middle), and AmB-treated CF human lung epithelia (right).
**Figure 5B** is a graph depicting ASL height in untreated normal human lung epithelial cells, untreated CF human lung epithelial cells; CF human lung epithelial cells treated with AmB added apically; CF human lung epithelial cells treated with C35deOAmB added apically; and CF human lung epithelial cells treated with AmB added basolaterally.
**Figure 5C** is a graph depicting ASL height in untreated normal human lung epithelial cells; untreated CF human lung epithelial cells; and CF human lung epithelial cells treated with AmB added in the concentrations shown.
**Figure 5D** is a graph depicting ASL height in untreated normal human lung epithelial cells; untreated CF human lung epithelial cells; and CF human lung epithelial cells treated with AmB added apically with or without basolateral addition of bumetanide.

### DETAILED DESCRIPTION OF THE INVENTION

Throughout the spectrum of living systems, robust protein networks comprised of pumps and channels drive targeted ions in targeted directions. In yeast, ATP-driven V-ATPase and Pma1 proton pumps in the vacuolar and plasma membranes, respectively, collaborate with passive Trk potassium channels in the plasma membrane to achieve the intracellular movement of potassium required for cell growth. Similarly, in human lung epithelia the Na⁺/K⁺-ATPase pump in the basolateral membrane generates the driving force for moving chloride ions into epithelial cells, and CFTR in the apical membrane mediates the passive release of these ions to the airway surface. This step, in turn, maintains airway surface hydration and thus mucociliary motion that protects against lung infections. Treacy, K. et al. Paediatr Child Health 21, 425-430 (2011).

Deficiencies of protein ion channels can cause dramatic phenotypes. Loss of Trk channels in yeast precludes cell growth, because the required uptake of environmental potassium is impaired. Loss of CFTR in humans causes the common and fatal genetic disease cystic fibrosis. The leading model of cystic fibrosis invokes that the apical release of intracellular chloride is compromised, leading to airway surface dehydration, loss of mucociliary motion, and chronic infections. Treacy, K. et al. Paediatr Child Health 21, 425-430 (2011). Importantly, in both of these cases the primary drivers of ion movement, the corresponding ATP-driven pumps, are still active. We thus hypothesized that a small molecule ion channel permeable to both potassium and chloride could collaborate with the respective protein ion pumps in each system to restore physiology.

Many small molecules with protein ion channel-like activity are known, and previous studies have demonstrated that such compounds can permeabilize planar lipid bilayers, liposomes, and/or cells. El-Etri, M. et al. Am. J. Physiol. 270, L386-392 (1996); Jiang, C. et al. Am. J. Physiol. Lung Cell. Mol. Physiol. 281, L1164-L1172 (2001); Koulov A.V et al. Angew. Chem. Int. Ed. Engl. 42, 4931-4933 (2003); Shen, B. et al. PloS One 7, e34694 (2012). However, the apical membranes of fully differentiated lung epithelia have many inherent barriers that might preclude small molecule-mediated permeabilization. Thus studies in model liposomes or cells do not necessarily translate to disease-relevant systems. Furthermore, it has not been previously determined whether imperfect mimicry of missing protein ion channels with small molecules can be sufficient to restore physiology, as judged by global phenotypic readouts, such as the growth of cells or normal mucociliary motion in epithelia.

However, in Ussing chamber experiments we tested several small molecules (N¹,N³-bis(((R)-1-(isobutylamino)-4-methyl-1-oxopentan-2-yl)oxy)isophthalamide (**2**), Methyl 3α-acetoxy-7α,12α-di[(4-nitrophenylaminocarbonyl)amino]-5β-cholan-24-oate (**3**), and Hydroxybisnorcholenic-spermine-sulfonate (**4**)) previously reported to enable chloride transport in liposomes and/or cells for the capacity to permeabilize differentiated human lung epithelia, but these compounds showed no such permeabilization, even at concentrations 10-fold higher than those used in previous reports.

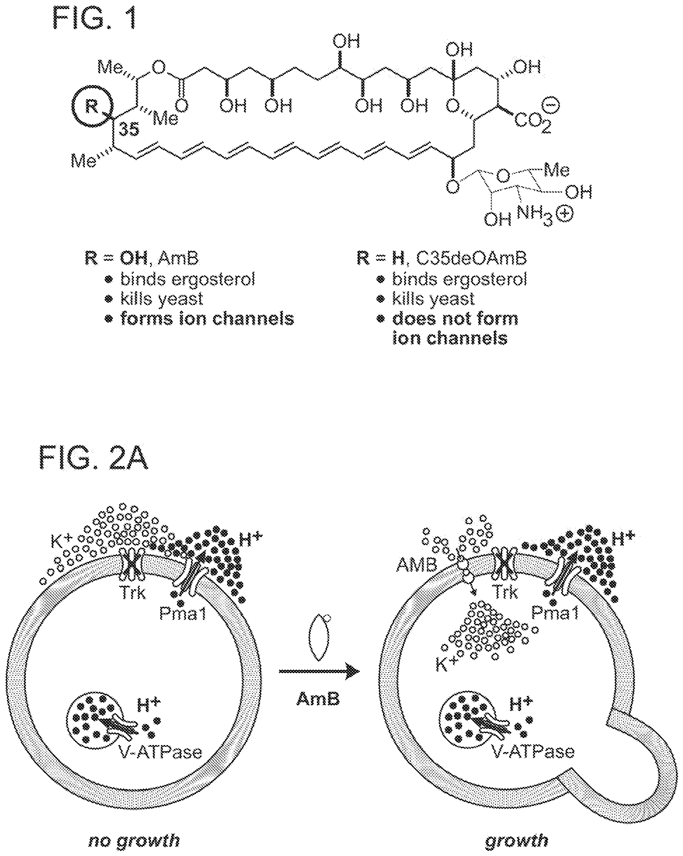

The ion channel-forming natural product amphotericin B (AmB) was identified as a small molecule probe to test our hypothesis. It can permeabilize both yeast cells and human lung epithelia, and AmB-based channels can conduct both potassium and chloride ions. Ermishkin, L.N. et al. Nature 262, 698-699 (1976); Ermishkin, L.N. et al. Biochim. Biophys. Acta 470, 357-367 (1977). AmB is also highly toxic, and this toxicity was thought to be inextricably linked to its membrane permeabilization. However, we found a synthesized derivative of AmB lacking a single oxygen atom at C35 (C35deOAmB) (Fig. 1) binds ergosterol but does not form ion channels, and yet still maintains potent fungicidal activity. Gray, K.C. et al. Proc. Natl. Acad. Sci. U.S.A. 109, 2234-2239 (2012). Further studies revealed that AmB primarily kills yeast by binding and extracting sterols from membranes, and is only cytotoxic when the amount of AmB exceeds that of ergosterol. Gray, K.C. et al. Proc. Natl. Acad. Sci. U.S.A. 109, 2234-2239 (2012); Anderson, T.M. et al. Nat. Chem. Biol. 10, 400-4006 (2014). Evidence supports the view that similar binding to cholesterol primarily causes toxicity in human cells. Wilcock, B.C et al. J. Am. Chem. Soc. 135, 8488-8491 (2013).

Therefore, we further hypothesized that the channel activity of AmB might be separated from its cytocidal activity by simply adding this compound at low concentrations. Guided by this logic, we found non-toxic nanomolar concentrations of AmB readily permeabilize both yeast cells and monolayers of differentiated human lung epithelia. The latter is especially remarkable because differentiated lung epithelia have barriers to small molecule-mediated permeabilization.

Importantly, C35deOAmB causes no such permeabilization, making this single atom-modified variant of AmB a unique and critical probe for determining whether any observed impacts of the natural product AmB on both yeast cells and human epithelia are specifically mediated by its ion channel activity.

An aspect of the invention is a method of treating a disease or condition characterized by decreased expression or reduced function of an ion channel, comprising administering to a subject in need thereof a therapeutically effective amount of a pore-forming polyene macrolide or pore-forming derivative thereof, thereby treating the disease or condition characterized by decreased expression or reduced function of the ion channel. The pore-forming polyene macrolide or pore-forming derivative thereof permits transmembrane trafficking of ions otherwise regulated by the ion channel.

Examples of diseases and conditions characterized by decreased expression or reduced function of an ion channel (sometimes referred to as channelopathies) include, without limitation, achromatopsia 2 (color blindness), achromatopsia 3, arrhythmogenic right ventricular dysplasia type 2, autosomal dominant (Thomsen) myotonia, autosomal dominant long-QT syndrome (Romano-Ward syndrome), autosomal dominant nocturnal frontal lobe epilepsy, autosomal dominant polycystic kidney disease (ADPKD), autosomal recessive (Becker) myotonia, autosomal recessive long-QT syndrome with deafness (Jervell-Lange-Nielsen syndrome), autosomal recessive retinitis pigmentosa, Bartter syndrome (renal salt loss, hypokalemic alkalosis), Bartter syndrome type III, Bartter syndrome type IV (associated with sensorineural deafness), BFNC (benign familial neonatal convulsions; epilepsy), BFNC (epilepsy) with myokymia, Brugada syndrome (idiopathic ventricular arrhythmia), calciumopathy, catecholaminergic polymorphic ventricular tachycardia, central core disease, childhood absence epilepsy, CMTX (X-linked Charcot-Marie-Tooth neuropathy), congenital bilateral aplasia of vas deferens, congenital hyperinsulinism, congenital insensitivity to pain, cystic fibrosis, Dent's disease (X-linked proteinuria and kidney stones), DFNA2 (dominant hearing loss), DFNA3 (autosomal dominant hearing loss), DFNB1 (autosomal recessive hearing loss), epilepsy, episodic ataxia, episodic ataxia with myokymia, erythromelalgia, familial atrial fibrillation, familial hemiplegic migraine, focal segmental glomerulosclerosis, generalized epilepsy with febrile and afebrile seizures, generalized epilepsy with febrile seizures plus (GEFS+), hyperekplexia (startle disease), hyperkalemic periodic paralysis, hypokalemic periodic paralysis, hypokalemic sensory overstimulation, hypomagnesimia with secondary hypocalcemia, juvenile myoclonus epilepsy, Liddle syndrome, Liddle syndrome (dominant hypertension), long-QT syndrome, long-QT syndrome with dysmorphic features (Andersen syndrome), maculopathy, malignant hyperthermia, mucolipidosis type IV, myasthenia congenital, myotonia congenital, nonsyndromic deafness, osteopetrosis (recessive or dominant), paramyotonia congenital, paroxysmal extreme pain syndrome, periodic paralysis, persistent hyperinsulinemic hypoglycemia of infancy (PHHI), potassium-aggravated myotonia, progressive familial heart block type I, pseudohypoaldosteronism type 1 (PHA1), retinitis pigmentosa, Rolandic epilepsy, short-QT syndrome, spinocerebellar ataxia type 6, spinocerebellar ataxia type 13, Timothy syndrome, and X-linked congenital stationary night blindness.

In certain embodiments, the disease or condition characterized by decreased expression or reduced function of an ion channel is selected from the group consisting of cystic fibrosis, hyperkalemic periodic paralysis, paramyotonia congenita, potassium aggravated myotonia, generalized epilepsy with febrile seizures plus (GEFS+), episodic ataxia, familial hemiplegic migraine, spinocerebellar ataxia type 13, long QT syndrome, Brugada syndrome, and mucolipidosis type IV.

In certain embodiments, the disease or condition characterized by decreased expression or reduced function of an ion channel is cystic fibrosis.

Examples of pore-forming polyene macrolides include, the following 36 structurally characterized mycosamine-containing polyene macrolides. 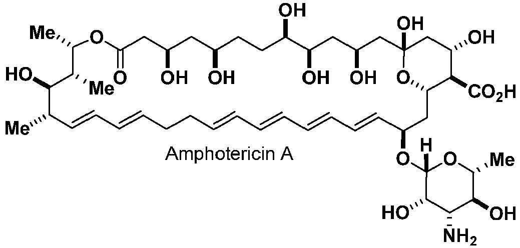 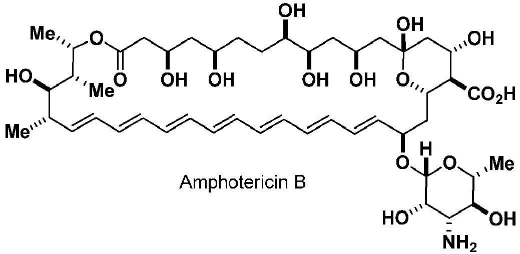 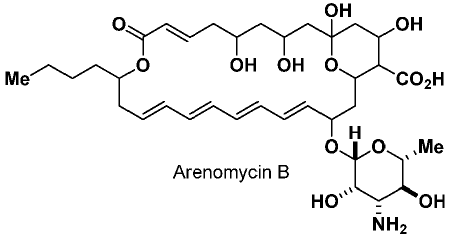 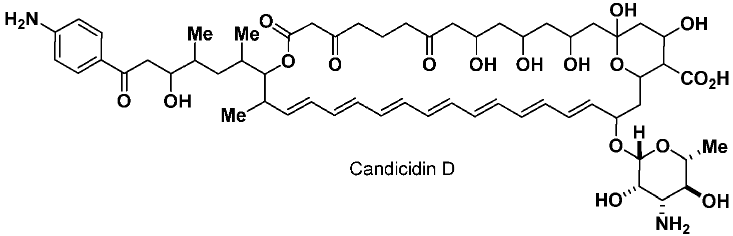 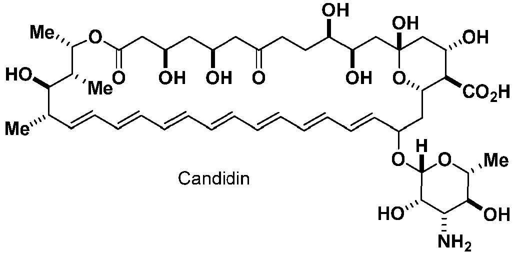 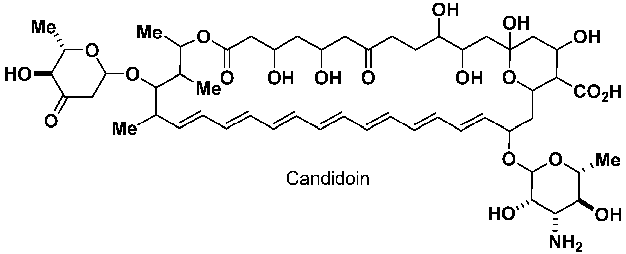 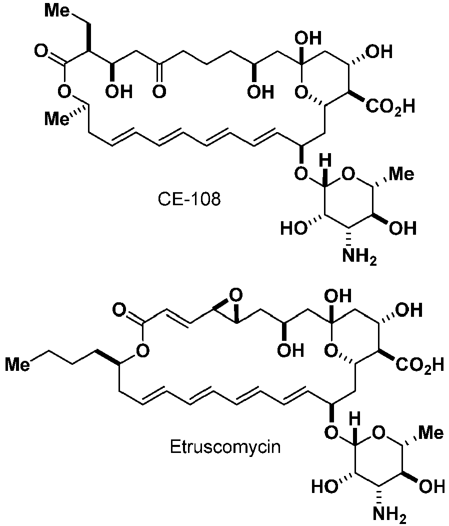 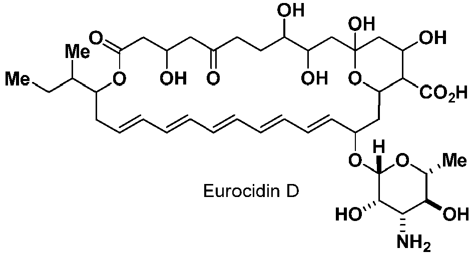 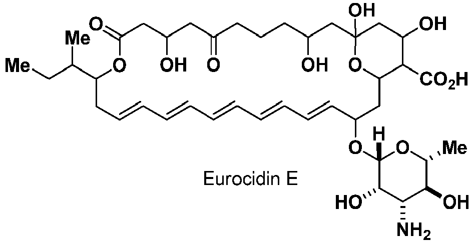 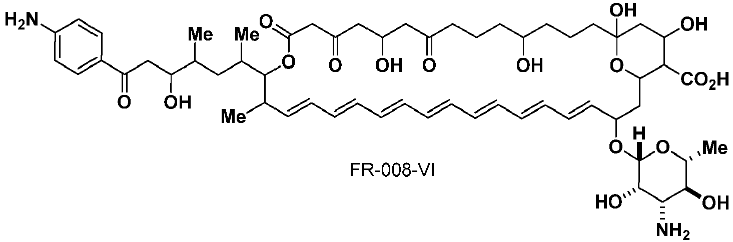 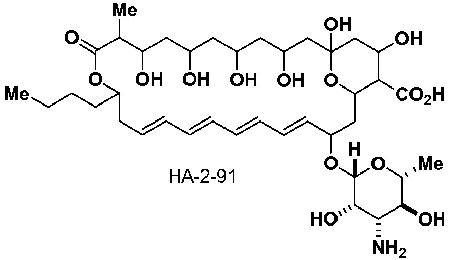 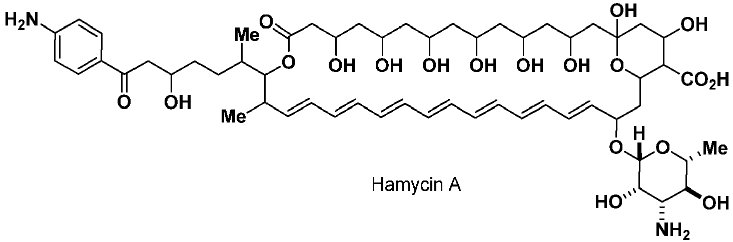 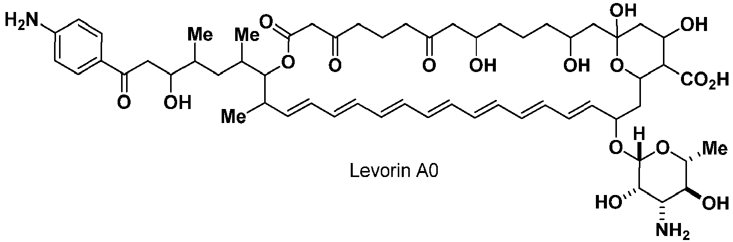 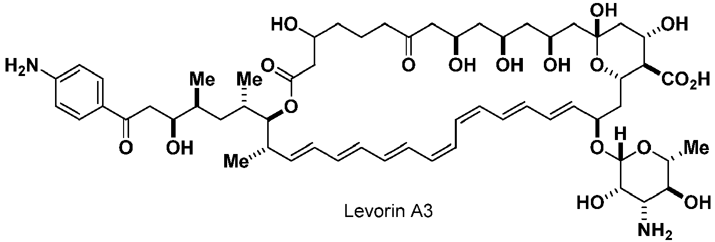 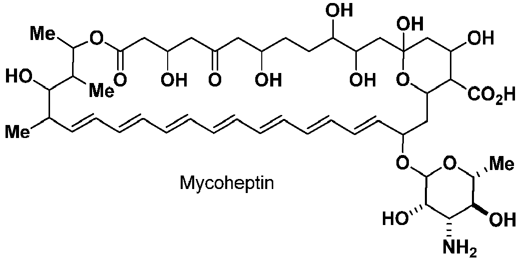 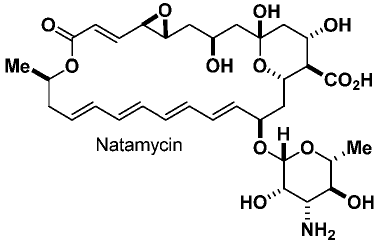 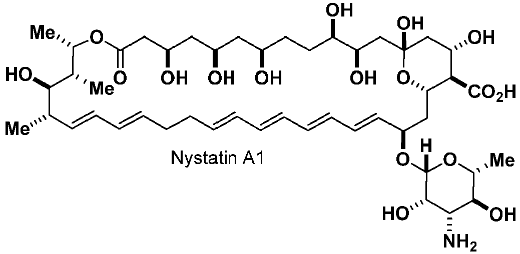 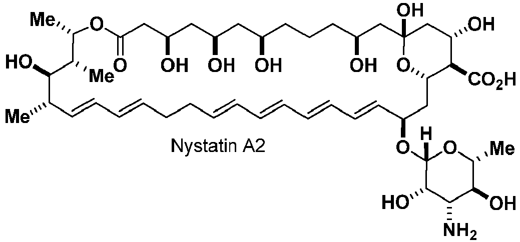 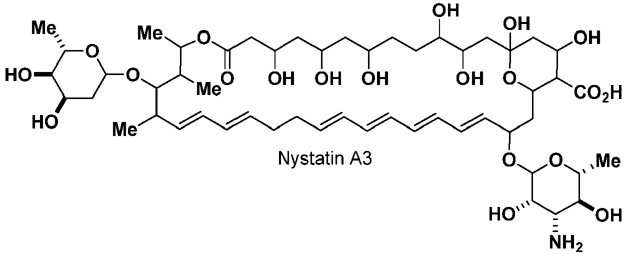 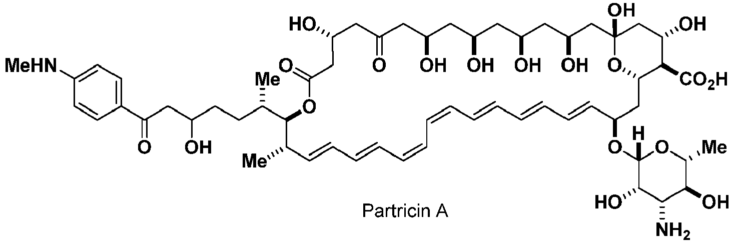 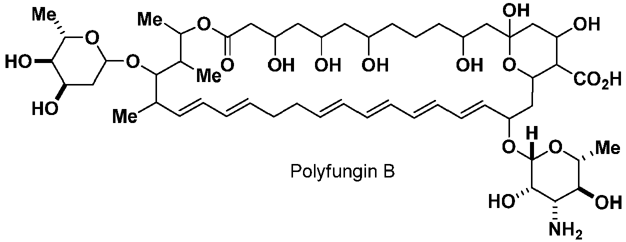 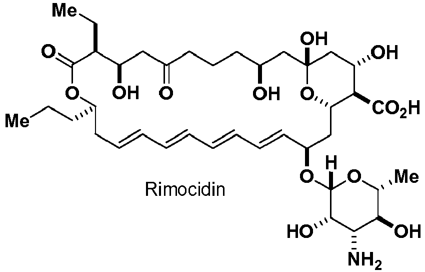 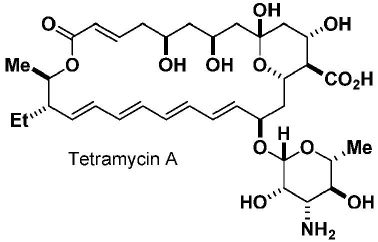 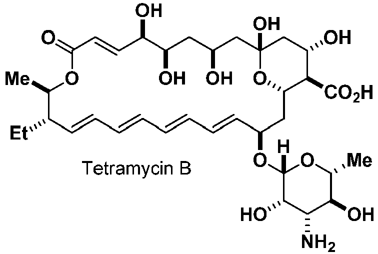 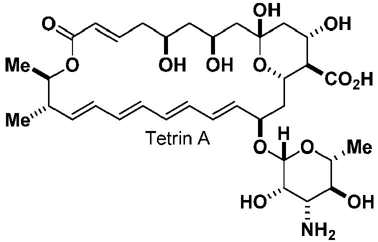 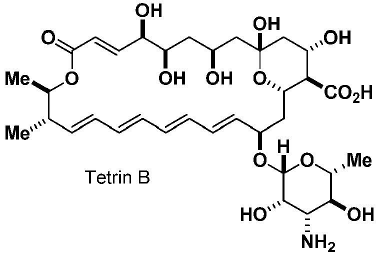 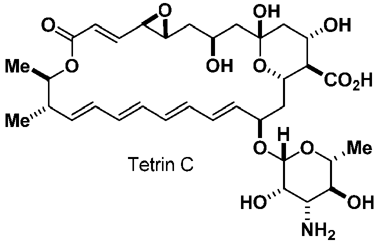 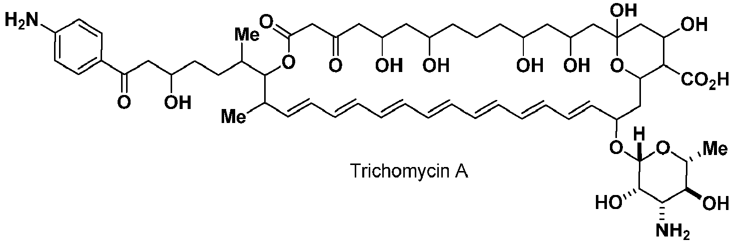 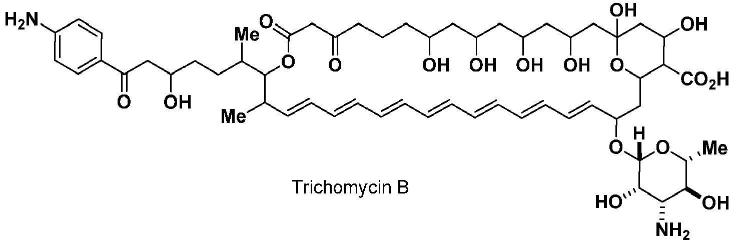 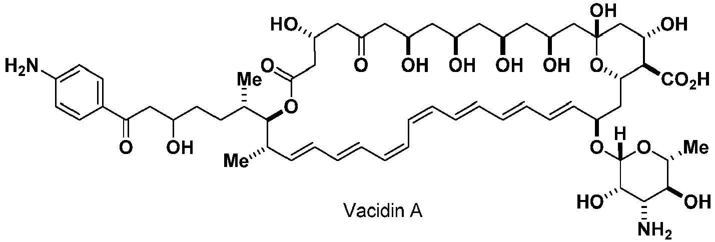 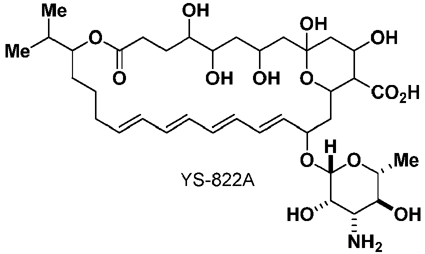 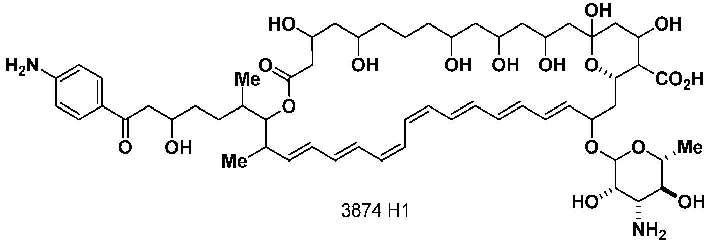 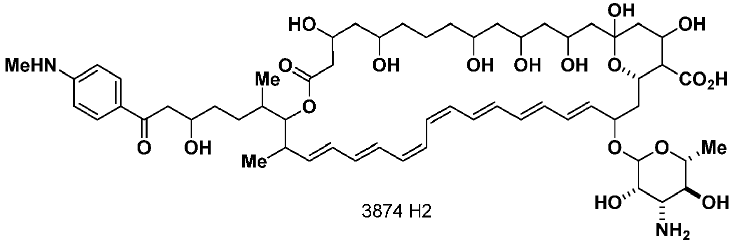 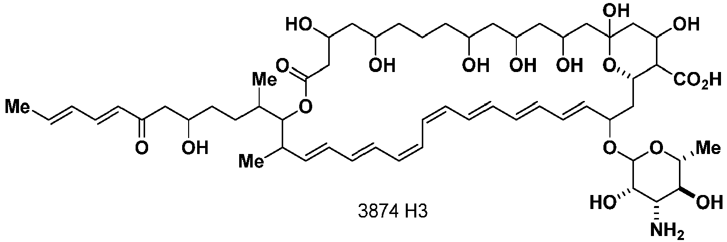 and

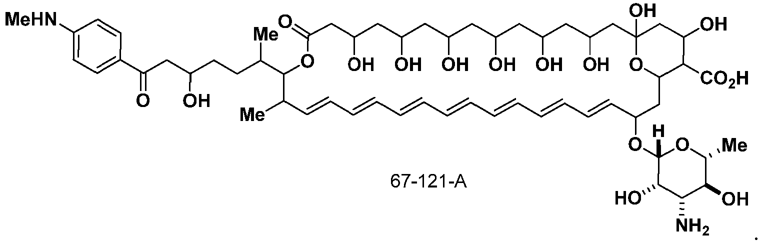

The following motif is 100% conserved in these compounds:

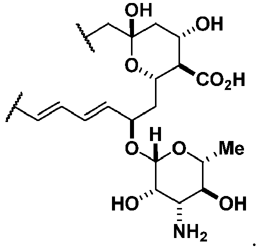

All spectroscopic and structure-activity evidence collected thus far supports the conclusion that the above highly conserved motif is the sterol binding domain of amphotericin B (i.e., the portion of the molecule that directly binds ergosterol and cholesterol).

In certain embodiments, the polyene macrolide is selected from the group consisting of amphotericin B (AmB), a nystatin (A1, A2, or A3), natamycin (also known as pimaricin), candicidin, and mepartricin, and any combination thereof.

In certain embodiments, the polyene macrolide is selected from the group consisting of amphotericin B (AmB), nystatin, natamycin, and any combination thereof.

In certain embodiments, the polyene macrolide is amphotericin B (AmB).

In certain embodiments, the subject does not have an infection treatable with the pore-forming polyene macrolide or pore-forming derivative thereof, e.g., the subject is not a subject having a yeast or fungal infection. For example, in certain embodiments, the subject does not have a yeast or fungal infection treatable with AmB.

In certain embodiments, the subject is not receiving the pore-forming polyene macrolide or pore-forming derivative thereof to treat an infection. For example, in certain embodiments, the subject is not receiving AmB to treat an infection.

In certain embodiments, the polyene macrolide is administered in a dose less than its minimum inhibitory concentration for *Saccharomyces cerevisiae.* As used herein, a "dose less than its minimum inhibitory concentration for *Saccharomyces cerevisiae"* refers to a dose sufficient to achieve a systemic or local concentration that is less than the minimum inhibitory concentration for *Saccharomyces cerevisiae.* In one embodiment, a "dose less than its minimum inhibitory concentration for *Saccharomyces cerevisiae*" refers to a dose sufficient to achieve a systemic concentration that is less than the minimum inhibitory concentration for *Saccharomyces cerevisiae.* In one embodiment, a "dose less than its minimum inhibitory concentration for *Saccharomyces cerevisiae*" refers to a dose sufficient to achieve a local concentration that is less than the minimum inhibitory concentration for *Saccharomyces cerevisiae.*

In certain embodiments, a "dose less than its minimum inhibitory concentration for *Saccharomyces cerevisiae*" refers to a dose sufficient to achieve a systemic or local concentration which is less than 90 percent of the minimum inhibitory concentration for *Saccharomyces cerevisiae.* In certain embodiments, a "dose less than its minimum inhibitory concentration for *Saccharomyces cerevisiae*" refers to a dose sufficient to achieve a systemic or local concentration which is less than 80 percent of the minimum inhibitory concentration for *Saccharomyces cerevisiae.* In certain embodiments, a "dose less than its minimum inhibitory concentration for *Saccharomyces cerevisiae*" refers to a dose sufficient to achieve a systemic or local concentration which is less than 70 percent of the minimum inhibitory concentration for *Saccharomyces cerevisiae.* In certain embodiments, a "dose less than its minimum inhibitory concentration for *Saccharomyces cerevisiae*" refers to a dose sufficient to achieve a systemic or local concentration which is less than 60 percent of the minimum inhibitory concentration for *Saccharomyces cerevisiae.* In certain embodiments, a "dose less than its minimum inhibitory concentration for *Saccharomyces cerevisiae*" refers to a dose sufficient to achieve a systemic or local concentration which is less than 50 percent of the minimum inhibitory concentration for *Saccharomyces cerevisiae.* In certain embodiments, a "dose less than its minimum inhibitory concentration for *Saccharomyces cerevisiae*" refers to a dose sufficient to achieve a systemic or local concentration which is less than 40 percent of the minimum inhibitory concentration for *Saccharomyces cerevisiae.* In certain embodiments, a "dose less than its minimum inhibitory concentration for *Saccharomyces cerevisiae*" refers to a dose sufficient to achieve a systemic or local concentration which is less than 30 percent of the minimum inhibitory concentration for *Saccharomyces cerevisiae.* In certain embodiments, a "dose less than its minimum inhibitory concentration for *Saccharomyces cerevisiae*" refers to a dose sufficient to achieve a systemic or local concentration which is less than 20 percent of the minimum inhibitory concentration for *Saccharomyces cerevisiae.* In certain embodiments, a "dose less than its minimum inhibitory concentration for *Saccharomyces cerevisiae*" refers to a dose sufficient to achieve a systemic or local concentration which is less than 10 percent of the minimum inhibitory concentration for *Saccharomyces cerevisiae.*

In certain embodiments, the pore-forming polyene macrolide or pore-forming derivative thereof is administered systemically.

In certain embodiments, the pore-forming polyene macrolide or pore-forming derivative thereof is administered locally.

In certain embodiments, the pore-forming polyene macrolide or pore-forming derivative thereof is administered to an airway of the subject. As used herein, "airway" refers to any conducting or respiratory epithelium of the respiratory tract. The term "airway" thus includes upper airways and lower airways, including nasal passages, paranasal sinuses, pharynx, larynx, trachea, bronchi, bronchioles, alveolar ducts, alveolar sacs, and alveoli.

In certain embodiments, the pore-forming polyene macrolide or pore-forming derivative thereof is administered as an aerosol to an airway of the subject.

As used herein, the terms "treating" and "treat" refer to performing an intervention that results in (a) preventing a condition or disease from occurring in a subject that may be at risk of developing or predisposed to having the condition or disease but has not yet been diagnosed as having it; (b) inhibiting a condition or disease, e.g., slowing or arresting its development; or (c) relieving or ameliorating a condition or disease, e.g., causing regression of the condition or disease. In one embodiment, the terms "treating" and "treat" refer to performing an intervention that results in (a) inhibiting a condition or disease, e.g., slowing or arresting its development; or (b) relieving or ameliorating a condition or disease, e.g., causing regression of the condition or disease.

A "yeast or fungal infection" as used herein refers to an infection with a yeast or fungus as defined herein.

As used herein, a "subject" refers to a living mammal. In various embodiments a subject is a non-human mammal, including, without limitation, a mouse, rat, hamster, guinea pig, rabbit, sheep, goat, cat, dog, pig, horse, cow, or non-human primate.

In certain embodiments, the subject is a human.

In certain embodiments, the subject is a human less than 12 years old.

In certain embodiments, the subject is a human at least 12 years old.

As used herein, a "subject having a yeast or fungal infection" refers to a subject that exhibits at least one objective manifestation of a yeast or fungal infection. In one embodiment a subject having a yeast or fungal infection is a subject that has been diagnosed as having a yeast or fungal infection and is in need of treatment thereof. Methods of diagnosing a yeast or fungal infection are well known and need not be described here in any detail.

As used herein, "administering" has its usual meaning and encompasses administering by any suitable route of administration, including, without limitation, intravenous, intramuscular, intraperitoneal, subcutaneous, direct injection (for example, into a tumor), mucosal, pulmonary (e.g., by inhalation or instillation (lavage)), oral, and topical.

In one embodiment, the administration is systemically administering.

In one embodiment, the administration is locally administering.

As used herein, the phrase "effective amount" refers to any amount that is sufficient to achieve a desired biological effect. A "therapeutically effective amount" is an amount that is sufficient to achieve a desired therapeutic effect, e.g., to treat a disease or condition characterized by decreased expression or reduced function of an ion channel.

Active pharmaceutical ingredients (APIs) in accordance with the invention can be combined with other therapeutic agents. The API and other therapeutic agent or agents may be co-administered simultaneously or sequentially. When the other therapeutic agent or agents are administered simultaneously, they can be administered in the same or separate formulations, but they are administered substantially at the same time as the API. The other therapeutic agent or agents are administered sequentially with one another and with the API when the administration of the other therapeutic agent or agents is temporally separated from the administration of the API. The separation in time between the administration of these compounds may be a matter of minutes or it may be longer.

Examples of other therapeutic agents include other antifungal agents, including AmB, as well as other antibiotics, anti-viral agents, anti-inflammatory agents, immunosuppressive agents, and anti-cancer agents.

As stated above, an "effective amount" refers to any amount that is sufficient to achieve a desired biological effect. Combined with the teachings provided herein, by choosing among the various active compounds and weighing factors such as potency, relative bioavailability, patient body weight, severity of adverse side-effects and preferred mode of administration, an effective prophylactic or therapeutic treatment regimen can be planned which does not cause substantial unwanted toxicity and yet is effective to treat the particular subject. The effective amount for any particular application can vary depending on such factors as the disease or condition being treated, the particular API being administered, the size of the subject, or the severity of the disease or condition. One of ordinary skill in the art can empirically determine the effective amount of a particular API and/or other therapeutic agent or agents without necessitating undue experimentation. It is preferred generally that a maximum dose be used, that is, the highest safe dose according to some medical judgment. Multiple doses per day may be contemplated to achieve appropriate systemic levels of compounds. Appropriate systemic levels can be determined by, for example, measurement of the patient's peak or sustained plasma level of the drug. "Dose" and "dosage" are used interchangeably herein.

Generally, daily intravenous doses of API, e.g., AmB, will be, for human subjects, similar to or less than usual daily intravenous doses of AmB. Similarly, daily other parenteral doses of API, e.g., AmB, will be, for human subjects, similar to or less than usual daily other parenteral doses of AmB.

In one embodiment, intravenous administration of an API may typically be from 0.1 mg/kg/day to 20 mg/kg/day. In one embodiment, intravenous administration of API may typically be from 0.2 mg/kg/day to 10 mg/kg/day. In one embodiment, intravenous administration of an API may typically be from 0.5 mg/kg/day to 5 mg/kg/day. In one embodiment, intravenous administration of an API may typically be from 1 mg/kg/day to 10 mg/kg/day. Intravenous dosing thus may be similar to, or advantageously, may be less than maximal tolerated doses of AmB.

Generally, daily oral doses of API will be, for human subjects, from about 0.01 milligrams/kg per day to 1000 milligrams/kg per day. It is expected that oral doses in the range of 0.5 to 50 milligrams/kg, in one or more administrations per day, will yield therapeutic results. Dosage may be adjusted appropriately to achieve desired drug levels, local or systemic, depending upon the mode of administration. For example, it is expected that intravenous administration would be from one order to several orders of magnitude lower dose per day. In the event that the response in a subject is insufficient at such doses, even higher doses (or effective higher doses by a different, more localized delivery route) may be employed to the extent that patient tolerance permits. Multiple doses per day are contemplated to achieve appropriate systemic levels of compounds.

For any compound described herein the therapeutically effective amount can be initially determined from animal models. A therapeutically effective dose can also be determined from human data for compounds of the invention which have been tested in humans and for compounds which are known to exhibit similar pharmacological activities, such as other related active agents (e.g., AmB). Higher doses may be required for parenteral administration. The applied dose can be adjusted based on the relative bioavailability and potency of the administered compound. Adjusting the dose to achieve maximal efficacy based on the methods described above and other methods as are well-known in the art is well within the capabilities of the ordinarily skilled artisan.

Formulations of the API are administered in pharmaceutically acceptable solutions, which may routinely contain pharmaceutically acceptable concentrations of salt, buffering agents, preservatives, compatible carriers, adjuvants, and optionally other therapeutic ingredients.

For use in therapy, an effective amount of the API can be administered to a subject by any mode that delivers the API to the desired location or surface. Administering the pharmaceutical composition of the API may be accomplished by any means known to the skilled artisan. Routes of administration include but are not limited to intravenous, intramuscular, intraperitoneal, intravesical (urinary bladder), oral, subcutaneous, direct injection (for example, into a tumor or abscess), mucosal (e.g., topical to eye), pulmonary (e.g., instillation or inhalation), and topical.

For intravenous and other parenteral routes of administration, the API, e.g., AmB, generally may be formulated similarly to AmB. For example, AmB can be formulated as a lyophilized preparation with desoxycholic acid, as a lyophilized preparation of liposome-intercalated or -encapsulated active compound, as a lipid complex in aqueous suspension, or as a cholesteryl sulfate complex. Lyophilized formulations are generally reconstituted in suitable aqueous solution, e.g., in sterile water or saline, shortly prior to administration.

For oral administration, the compounds (i.e., API and other therapeutic agent or agents) can be formulated readily by combining the active compound(s) with pharmaceutically acceptable carriers well known in the art. Such carriers enable the compounds of the invention to be formulated as tablets, pills, dragees, capsules, liquids, gels, syrups, slurries, suspensions and the like, for oral ingestion by a subject to be treated. Pharmaceutical preparations for oral use can be obtained as solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Optionally the oral formulations may also be formulated in saline or buffers, e.g., EDTA for neutralizing internal acid conditions or may be administered without any carriers.

Also specifically contemplated are oral dosage forms of the above component or components. The component or components may be chemically modified so that oral delivery of the derivative is efficacious. Generally, the chemical modification contemplated is the attachment of at least one moiety to the component molecule itself, where said moiety permits (a) inhibition of acid hydrolysis; and (b) uptake into the blood stream from the stomach or intestine. Also desired is the increase in overall stability of the component or components and increase in circulation time in the body. Examples of such moieties include: polyethylene glycol, copolymers of ethylene glycol and propylene glycol, carboxymethyl cellulose, dextran, polyvinyl alcohol, polyvinyl pyrrolidone and polyproline. Abuchowski and Davis, "Soluble Polymer-Enzyme Adducts", In: Enzymes as Drugs, Hocenberg and Roberts, eds., Wiley-Interscience, New York, N.Y., pp. 367-383 (1981); Newmark et al., J Appl Biochem 4:185-9 (1982). Other polymers that could be used are poly-1,3-dioxolane and poly-1,3,6-tioxocane. Preferred for pharmaceutical usage, as indicated above, are polyethylene glycol moieties.

For the component (or derivative) the location of release may be the stomach, the small intestine (the duodenum, the jejunum, or the ileum), or the large intestine. One skilled in the art has available formulations which will not dissolve in the stomach, yet will release the material in the duodenum or elsewhere in the intestine. Preferably, the release will avoid the deleterious effects of the stomach environment, either by protection of the API or by release of the biologically active material beyond the stomach environment, such as in the intestine.

To ensure full gastric resistance a coating impermeable to at least pH 5.0 is desirable. Examples of the more common inert ingredients that are used as enteric coatings are cellulose acetate trimellitate (CAT), hydroxypropylmethylcellulose phthalate (HPMCP), HPMCP 50, HPMCP 55, polyvinyl acetate phthalate (PVAP), Eudragit L30D, Aquateric, cellulose acetate phthalate (CAP), Eudragit L, Eudragit S, and shellac. These coatings may be used as mixed films.

A coating or mixture of coatings can also be used on tablets, which are not intended for protection against the stomach. This can include sugar coatings, or coatings which make the tablet easier to swallow. Capsules may consist of a hard shell (such as gelatin) for delivery of dry therapeutic (e.g., powder); for liquid forms, a soft gelatin shell may be used. The shell material of cachets could be thick starch or other edible paper. For pills, lozenges, molded tablets or tablet triturates, moist massing techniques can be used.

The therapeutic can be included in the formulation as fine multi-particulates in the form of granules or pellets of particle size about 1 mm. The formulation of the material for capsule administration could also be as a powder, lightly compressed plugs or even as tablets. The therapeutic could be prepared by compression.

Colorants and flavoring agents may all be included. For example, the therapeutic (or derivative) may be formulated (such as by liposome or microsphere encapsulation) and then further contained within an edible product, such as a refrigerated beverage containing colorants and flavoring agents.

One may dilute or increase the volume of the therapeutic with an inert material. These diluents could include carbohydrates, especially mannitol, α-lactose, anhydrous lactose, cellulose, sucrose, modified dextrans and starch. Certain inorganic salts may be also be used as fillers including calcium triphosphate, magnesium carbonate and sodium chloride. Some commercially available diluents are Fast-Flo, Emdex, STA-Rx 1500, Emcompress and Avicell.

Disintegrants may be included in the formulation of the therapeutic into a solid dosage form. Materials used as disintegrates include but are not limited to starch, including the commercial disintegrant based on starch, Explotab. Sodium starch glycolate, Amberlite, sodium carboxymethylcellulose, ultramylopectin, sodium alginate, gelatin, orange peel, acid carboxymethyl cellulose, natural sponge and bentonite may all be used. Another form of the disintegrants are the insoluble cationic exchange resins. Powdered gums may be used as disintegrants and as binders and these can include powdered gums such as agar, Karaya or tragacanth. Alginic acid and its sodium salt are also useful as disintegrants.

Binders may be used to hold the therapeutic agent together to form a hard tablet and include materials from natural products such as acacia, tragacanth, starch and gelatin. Others include methyl cellulose (MC), ethyl cellulose (EC) and carboxymethyl cellulose (CMC). Polyvinyl pyrrolidone (PVP) and hydroxypropylmethyl cellulose (HPMC) could both be used in alcoholic solutions to granulate the therapeutic.

An anti-frictional agent may be included in the formulation of the therapeutic to prevent sticking during the formulation process. Lubricants may be used as a layer between the therapeutic and the die wall, and these can include but are not limited to; stearic acid including its magnesium and calcium salts, polytetrafluoroethylene (PTFE), liquid paraffin, vegetable oils and waxes. Soluble lubricants may also be used such as sodium lauryl sulfate, magnesium lauryl sulfate, polyethylene glycol of various molecular weights, Carbowax 4000 and 6000.

Glidants that might improve the flow properties of the drug during formulation and to aid rearrangement during compression might be added. The glidants may include starch, talc, pyrogenic silica and hydrated silicoaluminate.

To aid dissolution of the therapeutic into the aqueous environment a surfactant might be added as a wetting agent. Surfactants may include anionic detergents such as sodium lauryl sulfate, dioctyl sodium sulfosuccinate and dioctyl sodium sulfonate. Cationic detergents which can be used and can include benzalkonium chloride and benzethonium chloride. Potential non-ionic detergents that could be included in the formulation as surfactants include lauromacrogol 400, polyoxyl 40 stearate, polyoxyethylene hydrogenated castor oil 10, 50 and 60, glycerol monostearate, polysorbate 40, 60, 65 and 80, sucrose fatty acid ester, methyl cellulose and carboxymethyl cellulose. These surfactants could be present in the formulation of the or derivative either alone or as a mixture in different ratios.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Microspheres formulated for oral administration may also be used. Such microspheres have been well defined in the art. All formulations for oral administration should be in dosages suitable for such administration.

For buccal administration, the compositions may take the form of tablets or lozenges formulated in conventional manner.

For administration by inhalation, the API may be conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g., gelatin for use in an inhaler or insufflator may be formulated containing a powder mix of the API and a suitable powder base such as lactose or starch.

Also contemplated herein is pulmonary delivery of the API. The API is delivered to the lungs of a mammal while inhaling and traverses across the lung epithelial lining, e.g., to the blood stream. Other reports of inhaled molecules include Adjei et al., Pharm Res 7:565-569 (1990); Adjei et al., Int J Pharmaceutics 63:135-144 (1990) (leuprolide acetate); Braquet et al., J Cardiovasc Pharmacol 13(suppl. 5):143-146 (1989) (endothelin-1); Hubbard et al., Annal Int Med 3:206-212 (1989) (α1-antitrypsin); Smith et al., 1989, J Clin Invest 84:1145-1146 (a-1-proteinase); Oswein et al., 1990, "Aerosolization of Proteins", Proceedings of Symposium on Respiratory Drug Delivery II, Keystone, Colorado, March, (recombinant human growth hormone); Debs et al., 1988, J Immunol 140:3482-3488 (interferon-gamma and tumor necrosis factor alpha) and Platz et al., U.S. Pat. No. 5,284,656 (granulocyte colony stimulating factor). A method and composition for pulmonary delivery of drugs for systemic effect is described in U.S. Pat. No. 5,451,569 (incorporated by reference), issued Sep. 19, 1995 to Wong et al.

Contemplated for use in the practice of this invention are a wide range of mechanical devices designed for pulmonary delivery of therapeutic products, including but not limited to nebulizers, metered dose inhalers, and powder inhalers, all of which are familiar to those skilled in the art.

Some specific examples of commercially available devices suitable for the practice of this invention are the Ultravent nebulizer, manufactured by Mallinckrodt, Inc., St. Louis, Mo.; the Acorn II nebulizer, manufactured by Marquest Medical Products, Englewood, Colo.; the Ventolin metered dose inhaler, manufactured by Glaxo Inc., Research Triangle Park, North Carolina; and the Spinhaler powder inhaler, manufactured by Fisons Corp., Bedford, Mass.

All such devices require the use of formulations suitable for the dispensing of API. Typically, each formulation is specific to the type of device employed and may involve the use of an appropriate propellant material, in addition to the usual diluents, adjuvants and/or carriers useful in therapy. Also, the use of liposomes, microcapsules or microspheres, inclusion complexes, or other types of carriers is contemplated. Chemically modified API may also be prepared in different formulations depending on the type of chemical modification or the type of device employed.

Formulations suitable for use with a nebulizer, either jet or ultrasonic, will typically comprise API dissolved in water at a concentration of about 0.1 to 25 mg of biologically active API per mL of solution. The formulation may also include a buffer and a simple sugar (e.g., for API stabilization and regulation of osmotic pressure). The nebulizer formulation may also contain a surfactant, to reduce or prevent surface induced aggregation of the API caused by atomization of the solution in forming the aerosol.

Formulations for use with a metered-dose inhaler device will generally comprise a finely divided powder containing the API suspended in a propellant with the aid of a surfactant. The propellant may be any conventional material employed for this purpose, such as a chlorofluorocarbon, a hydrochlorofluorocarbon, a hydrofluorocarbon, or a hydrocarbon, including trichlorofluoromethane, dichlorodifluoromethane, dichlorotetrafluoroethanol, and 1,1,1,2-tetrafluoroethane, or combinations thereof. Suitable surfactants include sorbitan trioleate and soya lecithin. Oleic acid may also be useful as a surfactant.

Formulations for dispensing from a powder inhaler device will comprise a finely divided dry powder containing API and may also include a bulking agent, such as lactose, sorbitol, sucrose, or mannitol in amounts which facilitate dispersal of the powder from the device, e.g., 50 to 90% by weight of the formulation. The API should advantageously be prepared in particulate form with an average particle size of less than 10 micrometers (µm), most preferably 0.5 to 5 µm, for most effective delivery to the deep lung.

Nasal delivery of API is also contemplated. Nasal delivery allows the passage of an API to the blood stream directly after administering the therapeutic product to the nose, without the necessity for deposition of the product in the lung. Formulations for nasal delivery include those with dextran or cyclodextran.

For nasal administration, a useful device is a small, hard bottle to which a metered dose sprayer is attached. In one embodiment, the metered dose is delivered by drawing the API in solution into a chamber of defined volume, which chamber has an aperture dimensioned to aerosolize and aerosol formulation by forming a spray when a liquid in the chamber is compressed. The chamber is compressed to administer the API. In a specific embodiment, the chamber is a piston arrangement. Such devices are commercially available.

Alternatively, a plastic squeeze bottle with an aperture or opening dimensioned to aerosolize an aerosol formulation by forming a spray when squeezed is used. The opening is usually found in the top of the bottle, and the top is generally tapered to partially fit in the nasal passages for efficient administration of the aerosol formulation. Preferably, the nasal inhaler will provide a metered amount of the aerosol formulation, for administration of a measured dose of the drug.

The APIs, when it is desirable to deliver them systemically, may be formulated for parenteral administration by injection, e.g., by bolus injection or continuous infusion. Formulations for injection may be presented in unit dosage form, e.g., in ampoules or in multi-dose containers, with an added preservative. The APIs may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the API in water-soluble form. Additionally, suspensions of the API may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethylcellulose, sorbitol, or dextran. Optionally, the suspension may also contain suitable stabilizers or agents which increase the solubility of the compounds to allow for the preparation of highly concentrated solutions.

Alternatively, the API may be in powder form for constitution with a suitable vehicle, e.g., sterile pyrogen-free water, before use.

The API may also be formulated in rectal or vaginal compositions such as suppositories or retention enemas, e.g., containing conventional suppository bases such as cocoa butter or other glycerides.

In addition to the formulations described above, the API may also be formulated as a depot preparation. Such long acting formulations may be formulated with suitable polymeric or hydrophobic materials (for example as an emulsion in an acceptable oil) or ion exchange resins, or as sparingly soluble derivatives, for example, as a sparingly soluble salt.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Suitable liquid or solid pharmaceutical preparation forms are, for example, aqueous or saline solutions for inhalation, microencapsulated, encochleated, coated onto microscopic gold particles, contained in liposomes, nebulized, aerosols, pellets for implantation into the skin, or dried onto a sharp object to be scratched into the skin. The pharmaceutical compositions also include granules, powders, tablets, coated tablets, (micro)capsules, suppositories, syrups, emulsions, suspensions, creams, drops or preparations with protracted release of active compounds, in whose preparation excipients and additives and/or auxiliaries such as disintegrants, binders, coating agents, swelling agents, lubricants, flavorings, sweeteners or solubilizers are customarily used as described above. The pharmaceutical compositions are suitable for use in a variety of drug delivery systems. For a brief review of methods for drug delivery, see Langer R, Science 249:1527-33 (1990).

The API and optionally other therapeutics may be administered *per se* (neat) or in the form of a pharmaceutically acceptable salt. When used in medicine the salts should be pharmaceutically acceptable, but non-pharmaceutically acceptable salts may conveniently be used to prepare pharmaceutically acceptable salts thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts can be prepared as alkaline metal or alkaline earth salts, such as sodium, potassium or calcium salts of the carboxylic acid group.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

Pharmaceutical compositions of the invention contain an effective amount of an API and optionally other therapeutic agent or agents included in a pharmaceutically acceptable carrier. The term "pharmaceutically acceptable carrier" means one or more compatible solid or liquid filler, diluents or encapsulating substances which are suitable for administration to a human or other vertebrate animal. The term "carrier" denotes an organic or inorganic ingredient, natural or synthetic, with which the active ingredient is combined to facilitate the application. The components of the pharmaceutical compositions also are capable of being commingled with the compounds of the present invention, and with each other, in a manner such that there is no interaction which would substantially impair the desired pharmaceutical efficiency.

The therapeutic agent(s), including specifically but not limited to the API, may be provided in particles. Particles as used herein means nanoparticles or microparticles (or in some instances larger particles) which can consist in whole or in part of the API or the other therapeutic agent(s) as described herein. The particles may contain the therapeutic agent(s) in a core surrounded by a coating, including, but not limited to, an enteric coating. The therapeutic agent(s) also may be dispersed throughout the particles. The therapeutic agent(s) also may be adsorbed into the particles. The particles may be of any order release kinetics, including zero-order release, first-order release, second-order release, delayed release, sustained release, immediate release, and any combination thereof, etc. The particle may include, in addition to the therapeutic agent(s), any of those materials routinely used in the art of pharmacy and medicine, including, but not limited to, erodible, nonerodible, biodegradable, or nonbiodegradable material or combinations thereof. The particles may be microcapsules which contain the API in a solution or in a semi-solid state. The particles may be of virtually any shape.

Both non-biodegradable and biodegradable polymeric materials can be used in the manufacture of particles for delivering the therapeutic agent(s). Such polymers may be natural or synthetic polymers. The polymer is selected based on the period of time over which release is desired. Bioadhesive polymers of particular interest include bioerodible hydrogels described in Sawhney H S et al. (1993) Macromolecules 26:581-7, the teachings of which are incorporated herein. These include polyhyaluronic acids, casein, gelatin, glutin, polyanhydrides, polyacrylic acid, alginate, chitosan, poly(methyl methacrylates), poly(ethyl methacrylates), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate).

The therapeutic agent(s) may be contained in controlled release systems. The term "controlled release" is intended to refer to any drug-containing formulation in which the manner and profile of drug release from the formulation are controlled. This refers to immediate as well as non-immediate release formulations, with non-immediate release formulations including but not limited to sustained release and delayed release formulations. The term "sustained release" (also referred to as "extended release") is used in its conventional sense to refer to a drug formulation that provides for gradual release of a drug over an extended period of time, and that preferably, although not necessarily, results in substantially constant blood levels of a drug over an extended time period. The term "delayed release" is used in its conventional sense to refer to a drug formulation in which there is a time delay between administration of the formulation and the release of the drug there from. "Delayed release" may or may not involve gradual release of drug over an extended period of time, and thus may or may not be "sustained release."

Use of a long-term sustained release implant may be particularly suitable for treatment of chronic conditions. "Long-term" release, as used herein, means that the implant is constructed and arranged to deliver therapeutic levels of the active ingredient for at least 7 days, and preferably 30-60 days. Long-term sustained release implants are well-known to those of ordinary skill in the art and include some of the release systems described above.

### EXAMPLES

Having now described the present invention in detail, the same will be more clearly understood by reference to the following examples.

### General Materials and Methods

*Yeast cell lines and growth conditions.* Wild type *S. cerevisiae* (ATCC 9763), and trk1Δtrk2Δ *S. cerevisiae* (SGY 1528) were maintained with yeast peptone adenine dextrose (YPAD) growth media consisting of 10 g/L yeast extract, 20 g/L peptone, 20 g/L dextrose, 0.015 g/L adenine hemisulfate salt (final potassium concentration = 10 mM). For solid media, 20 g/L agar was added to this same mixture. To culture trk1Δtrk2Δ *S. cerevisiae,* additional potassium was added as potassium chloride (100 mM KCl). The media was adjusted to pH 5.0 using citric acid. After autoclave sterilization, dextrose was subsequently added as a sterile 20% w/v solution in water (dextrose solutions were filter-sterilized using a 0.22 µm filter). Liquid cultures were incubated at 30 °C on a rotary shaker (200 rpm). Solid cultures were maintained at 30 °C in an incubator. For solid media containing AmB (AK Scientific), the media was allowed to cool for 15 minutes before addition of AmB at a final concentration of 125 nM. AmB was added to the media as a solution in DMSO. Plates were examined for growth after 24-48 hours of incubation.

*Disc diffusion assay.* The assay was performed as previously described. National Committee for Clinical Laboratory Standards in *Performance Standards for Antimicrobial Disk Susceptibility Tests; M2-A8 Approved Standard* (NCCLS, Wayne) (2003).

*Broth microdilution minimum inhibitory concentration (MIC) assay.* The protocol for the MIC broth microdilution assay was adapted from the Clinical and Laboratory Standards Institute document M27-A2 and performed as previously described. Gray, K.C. et. al. Proc. Natl. Acad. Sci. U.S.A. 109, 2234-2239 (2012); Palacios, D.S. et al. J. Am. Chem. Soc. 129, 13804-13805 (2007).

*Functional complementation of a protein with a small molecule assay.* The protocol for the rescue broth microdilution assay was conducted as described in the MIC assay above with the following additions: trk1Δtrk2Δ yeast were grown in high potassium (100 mM) YPAD liquid media overnight and transferred to normal potassium YPAD liquid media containing vehicle or the indicated concentration of AmB.

*Tetraethylammonium block assay.* The protocol for the tetraethylammonium block assay was conducted as described in the functional complementation assay above with the addition of 125 nM AmB and the indicated concentration of tetraethylammonium to the normal potassium YPAD media and the indicated concentration of tetraethylammonium to the high potassium (100 mM) YPAD media.

*⁸⁶Rb*⁺ *uptake assay.* The procedure for ⁸⁶Rb⁺ uptake was adapted from Mulet, J.M. et al. Mol. Cell. Biol. 19, 3328-3337 (1999). Overnight yeast cultures were grown as described above. The supernatant was poured off, and the cells were resuspended in sterile water. This wash was repeated two more times. The cells were then resuspended in 40 mL of the potassium starvation/uptake buffer (50 mM succinic acid, 2% glucose, adjusted to pH 5.5 with Tris). After 3 hour incubation in the potassium starvation buffer, cells were centrifuged and washed twice with sterile water. Cells were resuspended in 1 mL of the potassium starvation/uptake buffer. Cell concentration was determined using an INCYTO® Neubauer disposable hemocytometer, and wild type *S. cerevisiae* and trk1Δtrk2Δ cells were diluted to the same cell density. 700 µL of the yeast suspension was added to a 1.5 mL Eppendorf tube. After a 5 minute pre-incubation in the potassium starvation/uptake medium, AmB or C35deOAmB was added (final concentration 3 µM) as a DMSO solution. ⁸⁶RbCl (1.1 µCi) was then added to the reaction mixtures. Reaction mixtures were vortexed to ensure a homogeneous solution. At the indicated times, the uptake reaction was stopped by taking a 100 µL aliquot from the reaction mixture and diluting with 10 mL of ice-cold 20 mM MgCl₂. Cells were then collected via vacuum filtration through a 0.45-µm-pore-size nitrocellulose filter (Millipore HAWP). Cells were washed with two 15 mL aliquots of 20 mM ice-cold MgCl₂. Moist filters were transferred to plastic vials for measuring radioactivity. Radioactivity was monitored using a Perkin Elmer Wizard automatic gamma counter. Results are reported in counts per minute as an average of three biological replicates.

*Cell viability assay.* WT and trk1Δtrk2Δ cells were inoculated in high potassium (100 mM KCl) YPAD. Starter cultures were grown at 30 °C for 14-15 hours. Cells were then cultured in high potassium YPAD at 30 °C for 2-2.5 hours. 100 µL of a 0.25 mg/mL propidium iodide (PI, Sigma-Aldrich P4864) solution was prepared. After 2-2.5 hours, cells were centrifuged at 800g for 5 minutes, at 23 °C. The supernatant was decanted, cells were resuspended in 40 mL of high potassium YPAD, vortexed, and centrifuged. The wash step was repeated. After pouring off the supernatant, cells were resuspended in 15 mL of high potassium YPAD, vortexed, and diluted to an OD₆₀₀ of 0.5. To set one (WT, WT + PI, trk1Δtrk2Δ, and trk1Δtrk2Δ + PI) and set two (WT + PI + AmB, trk1Δtrk2Δ + PI + AmB), 26.4 µL of DMSO were added to 1 mL aliquots of WT and trk1Δtrk2Δ, and incubated at 30 °C for 30 minutes. A 1% w/v low gelling temperature agarose (Sigma-Aldrich A9414) was prepared in normal potassium (10 mM) YPAD. When set one incubation completed, cells were pulse centrifuged, supernatant was removed, and resuspended in normal potassium YPAD to wash. Cells were centrifuged and resuspended in 250 µL of normal potassium YPAD. Afterwards, 1 µL of 0.25 mg/mL PI dye was added to appropriate samples and 250 µL of 1% agarose was added. Samples were plated onto microscope slides with cover slips and were incubated in humidity chambers for 24 hours at 30 °C. For set two, cells were resuspended in normal potassium YPAD containing 125 nM AmB instead, to wash. Cells were resuspended in 250 µL of normal potassium YPAD containing 0.250 µM AmB before adding to 1% agarose (to obtain 125 nM AmB final). Confocal microscope (Zeiss LSM700) images were taken at 40x where ∼15 random images were taken per slide, per treatment group, per experiment. The total number of PI stained cells was subtracted from the total cells recorded, and divided by total cells to give % viability. At least 200 cells were recorded per treatment group and 4 independent sets of data were obtained.

*Sustainable restoration of cell growth assay*. Wild type and trk1Δtrk2Δ yeast were treated as described in the functional complementation experiment described above, with the trk1Δtrk2Δ yeast treated with 125 nM AmB. The OD₆₀₀ was measured every hour for 24 hours, including a reading at time zero. Wild type yeast were then streaked onto normal potassium YPAD agar plates and AmB-rescued Trk1Δtrk2Δ yeast were streaked onto AmB-containing (0.125 µM) normal potassium YPAD agar plates. These agar plates were then incubated at 30 °C for about 48 hours. The same procedure was then repeated for over 42 days, measuring the max OD₆₀₀ and doubling time every three days. Doubling time was determined using the following equation: T_{d} = (t₂-t₁) x [log(2)/log (q₂/q₁)], where t₂ and t₁ represent the time at the two points and q₂ and q₁ represent the OD₆₀₀ values in the exponential phase of growth (OD₆₀₀ from 0.2 to 0.6).

*Probing sensitivities to known chemical inhibitors assay*. Overnight cultures were grown as described in the MIC assay. Prior to harvesting cells, small molecules AmB, nystatin A1 (Riedel-de-Haen), candicidin (TOKU-E), or mepartricin (Santa Cruz Biotechnology) and chemical inhibitors nocodazole (Sigma-Aldrich), ebselen (Cayman Chemical), or bafilomycin B1 (Santa Cruz Biotechnology were prepared as stock solutions in DMSO. Saturated cell cultures were centrifuged for 5 minutes at 1000 x g. The supernatant was poured off, and cells were resuspended in sterile MilliQ water. Cells were centrifuged again, and supernatant was poured off. The wash step was repeated. The supernatant was poured off, and cells were resuspended in normal potassium YPAD. Cells were diluted with normal potassium YPAD to an OD₆₀₀ of 0.01. Next, an appropriate volume of AmB, nystatin A1, candicidin, or mepartricin was added to give a final rescuing concentration of 125 nM, 1000 nM, 8 nM, and 8 nM, respectively. A control was prepared by adding the corresponding volume of DMSO to trk1Δtrk2Δ cells. 195 µL of WT + small molecule, 195 µL of trk1Δtrk2Δ + small molecule, and 195 µL of trk1Δtrk2Δ + DMSO were added to a 96 well plate. Next, 5 µL of DMSO or chemical inhibitor was added to each well, with each concentration tested in triplicate for both WT and trk1Δtrk2Δ. The plate was covered and incubated at 30 °C for 24 hours. A BioTek Synergy H1 Hybrid Reader was used to measure the OD₆₀₀. Utilizing GraphPad PRISM, data were fitted by nonlinear regression, inhibition dose response, variable slope (four parameters) to yield EC50 values with SEM. For statistical analysis, EC₅₀ values from the two treatment groups were compared by unpaired t-test.

*Human cell lines and growth conditions.* NuLi and CuFi-1 cells were cultured at the air-liquid interface as previously described, using a 1:1 mixture of DMEM:Ham's F-12 supplemented with 2% v/v Ultroser G (Crescent Chemical). Zabner, J. et al. Am. J. Physiol. Lung Cell. Mol. Physiol. 284, L844-L854 (2003). The membrane supports used were Millicell 0.4 µm PCF inserts (Millipore PIHP01250) for Ussing chamber studies, Corning Costar 0.4 µm Transwell Clear Polyester Membrane inserts (Corning 3470) for rotational mucus transport studies, and Millicell 1.0 µm PCF inserts (Millipore PIRP15R48) for ASL studies. These membranes were allowed to mature at an air-liquid interface for a minimum of 14 days to reach full differentiation.

*Synthesis and characterization of ionophores*. Compounds N¹,N³-bis(((R)-1-(isobutylamino)-4-methyl-1-oxopentan-2-yl)oxy)isophthalamide **(2)**, Methyl 3α-acetoxy-7α,12α-di[(4-nitrophenylaminocarbonyl)amino]-5β-cholan-24-oate **(3)**, and Hydroxybisnorcholenic-spermine-sulfonate **(4)** were synthesized as previously described and purified by preparative HPLC. Jiang, C. et. al. Am. J. Physiol. Lung Cell. Mol. Physiol. 281, L1164-L1172 (2001); Deng, G. et al. J. Am. Chem. Soc. 118, 8975-8976 (1996); Sadownik, A. et al. J. Am. Chem. Soc. 117, 6138-6139 (1995); Davis, A.P. et al. Synlett S1, 991-993 (1999); del Amo, V. et al. Org. Biomol. Chem. 2, 3320-3328 (2004); Koulov, A.V. et al. Angew. Chem. Int. Ed. Engl. 42, 4931-4933 (2003); Li, X. et al. J. Am. Chem. Soc. 129, 7264-7265 (2007); Shen, B. et al. PloS One 7, e34694 (2012).

*Ussing chamber studies.* Experiments were adapted from procedures described by Zabner, J. et al. Am. J. Physiol. Lung Cell. Mol. Physiol. 284, L844-L854 (2003). Mature, differentiated CuFi membranes grown on Millicell 0.4 µm PCF inserts were mounted in a dual-channel Ussing chamber (Warner U2500) using the culture cup insert for Millicell adapter, 12 mm (U9924M-12). Stock solutions of 100 mM amiloride and 4,4'-diisothiocyano-2,2'-stilbenedisulfonic acid (DIDS) were prepared in DMSO. Stocks of the experimental solutions of AmB, C35deOAmB, and **1-4** were prepared in DMSO 1000x more concentrated than the desired final concentration in buffer. Electrodes were prepared with 3.5% agar and 3 M KCl. 5 mL of 37 °C high chloride buffer (135 mM NaCl, 5 mM HEPES, 2.4 mM K₂HPO₄, 0.6 mM KH₂PO₄, 1.2 mM CaCl₂, 1.2 mM MgCl₂, 5 mM dextrose, pH 7.4 with 10 N NaOH) was placed on both sides of the chamber, kept at 37 °C and gassed with compressed air. Epithelial sodium channel (ENaC) and calcium-activated chloride channel (CaCC) were inhibited by 5 µL of amiloride and DIDS, respectively, to achieve a baseline for small molecule-mediated permeabilization. To establish a basolateral (135 mM) to apical (4.9 mM) chloride gradient, the buffer on the apical side of the membrane was replaced by 5 mL of low chloride buffer (135 mM sodium gluconate, 5 mM HEPES, 2.4 mM K₂HPO₄, 0.6 mM KH₂PO₄, 1.2 mM CaCl₂, 1.2 mM MgCl₂, 5 mM dextrose, pH 7.4 with 10 N NaOH). When baseline was stabilized, 5 µL of the small molecule for testing was added to the apical buffer and then short circuit current was monitored for at least 10 minutes. Area under the curve of short circuit current traces for 15 minutes of data after compound addition was reported. These values were averaged across triplicate runs and reported with standard error of the mean.

*Airway surface liquid (ASL) height studies.* ASL height was studied using an established fluorescent dye assay. Worthington, E.N. et al. Methods Mol. Biol. 742, 77-92 (2011); Myerburg, M.M. et al. Am. J. Respir. Cell. Mol. Biol. 42, 676-684 (2010). Mature, differentiated membranes were grown on Millicell 0.1 µm polyethylene terephthalate hanging cell culture inserts. Wild type (NuLi) lung epithelia were treated with perfluorocarbon (FC-72) vehicle, and CuFi epithelia were treated with vehicle or 500 nM AmB and incubated for 24 hours at 37 °C. On the day of imaging, 2.5 µL of a 70 kDa Texas Red-dextran conjugate (Molecular Probes) solution in PBS was added to the apical side of the membranes, followed by 100 µL of FC-770 to prevent evaporation. These were placed on 100 µL of PBS on a 10 mm glass bottom Fluorodish for imaging (World Precision Instruments). Membranes were imaged immediately after dye addition and again at 24 hours to examine dye absorption. Three Z-stack images per membrane in biological n = 6 were taken on an Zeiss LSM700 confocal microscope at 40x oil immersion. These images were analyzed using ImageJ to determine the area of fluorescence in the left hand 200-micron width of each image. Images were converted to 8-bit, Gaussian Blur was applied, and made binary. The parameters for Analyze Particles were particles from 1-Infinity µm² in size and from 0%-100% circularity.

### Example 1: AmB Restores Cell Growth in Potassium Channel-Deficient Yeast

We first tested the hypothesis that AmB could restore cell growth in potassium channel-deficient yeast by using a modified functional complementation experiment. Lee, M.G. et al. Nature 327, 31-35 (1987). Relative to human epithelia, yeast represent a simple, well-defined, and experimentally tractable eukaryotic model organism in which the major players that mediate transmembrane ion transport are known and relatively well understood. We specifically focused on yeast lacking Trk potassium ion transporters (trk1Δtrk2Δ) which cannot grow in the presence of normal extracellular potassium concentrations (10 mM). Ko, C.H. et al. Mol. Cell. Biol. 11, 4266-4273 (1991). The Trk proteins are potassium selective, inwardly rectified, and extensively regulated, making them a challenging target for perfect functional replication. However, yeast also have proton pumps Pma1 and V-ATPase in the plasma and vacuolar membranes, respectively, which collaborate with Trk ion channels by creating an electrochemical driving force for physiological transmembrane potassium movement. Yeast also possess multiple mechanisms for addressing the lack of selectivity of AmB for other ions, such as dedicated efflux pumps that selectively export sodium. We thus hypothesized that in Trk-deficient yeast, Pma1 and V-ATPase might collaborate with AmB-based ion channels to collectively restore physiological transmembrane potassium transport and, therefore, cell growth.

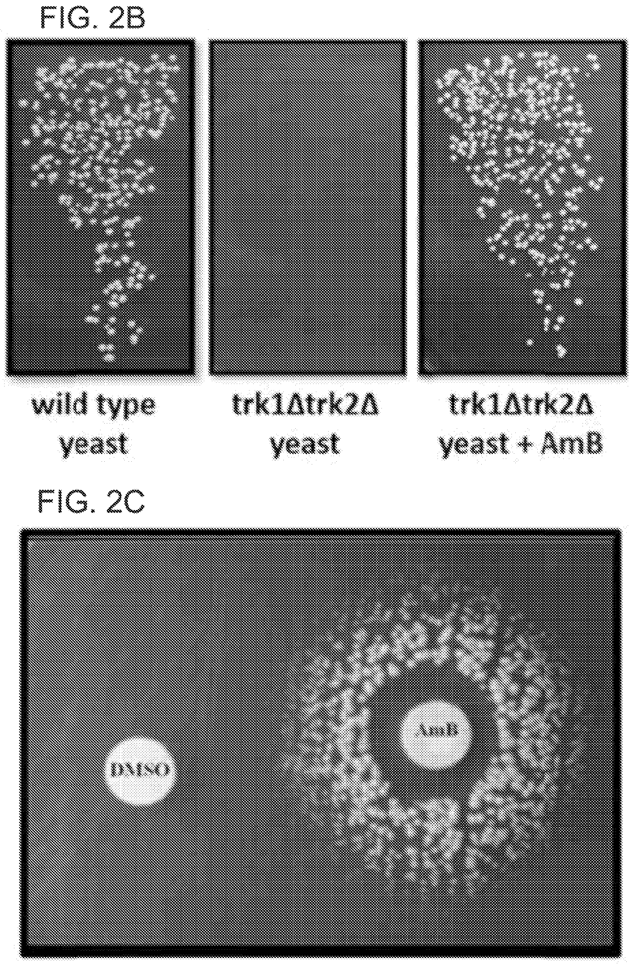

Consistent with prior reports (Minor, D.L. et al. Cell 96, 879-891 (1999)), growth was observed when wild type *Saccharomyces cerevisiae* were streaked onto agar plates containing normal concentrations of potassium (Fig. 2B, left), and no growth was observed for potassium channel-deficient strain trk1Δtrk2Δ (Fig. 2B, middle). Strikingly, the addition of a low concentration of AmB (125 nM) to the agar plate restored vigorous growth of the trk1Δtrk2Δ mutant (Fig. 2B, right).

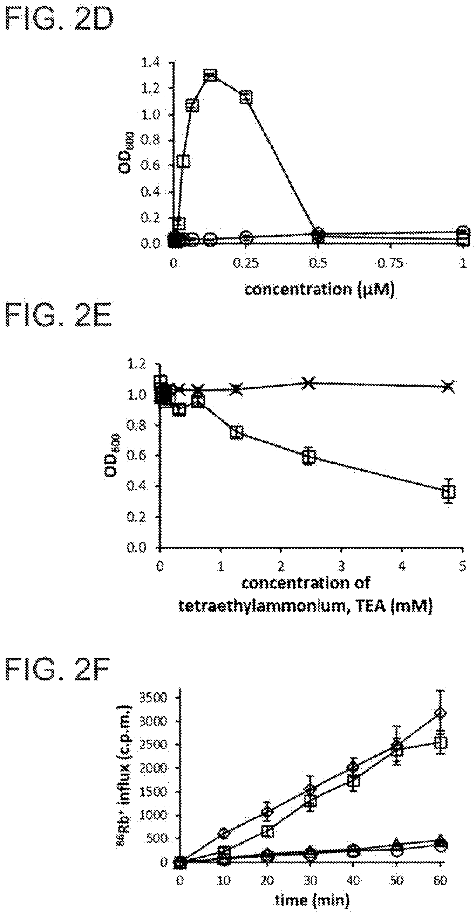

A series of additional experiments confirmed the observed restoration of cell growth is caused by small molecule-based ion channel activity. A disc diffusion assay visually revealed the predicted dependence of the growth rescue on the concentration of AmB (Fig. 2C). To quantify the concentration dependence and eliminate the potentially complicating issue of plating efficiency, we also measured trk1Δtrk2Δ yeast cell growth in a broth dilution assay (Fig. 2D). Consistent with the disc diffusion results, no cell growth was observed in the absence of AmB, a dose-dependent increase of growth was observed at intermediate concentrations, and no growth was observed at or above the minimum inhibitory concentration of this antifungal agent. Further ruling out any type of generic hormetic effects, no growth stimulatory effects were observed when wild type cells were treated with AmB.

### Example 2: Channel-Inactivated Variant C35deOAmB Does Not Restore Cell Growth in Potassium Channel-Deficient Yeast

To probe directly the importance of the ion channel activity of AmB, we also tested the channel-inactivated variant C35deOAmB (Fig. 1). This derivative failed to restore growth in trk1Δtrk2Δ cells at any tested concentration (Fig. 2D).

### Example 3: Sterically Bulky Tetraethylammonium Cation Blocks Functional Complementation Observed with AmB

In a complementary experiment, we utilized the sterically bulky tetraethylammonium cation to block the AmB-based ion channel²⁴. Borisova, M.P. et al. Biochim. Biophys. Acta 553, 450-459 (1979). This cation inhibited the functional complementation observed with AmB in a dose-dependent manner without causing general toxicity (Fig. 2E).

### Example 4: AmB, But Not Channel-Inactivated Variant C35deOAmB, Restores ⁸⁶Rb⁺ Uptake in Potassium Channel-Deficient Yeast

We further monitored uptake of radioactive ⁸⁶Rb⁺ as a reporter of transmembrane potassium movement (Fig. 2F). ⁸⁶Rb⁺ uptake was observed in wild type yeast but not in the trk1Δtrk2Δ mutant, and no uptake was observed when the trk1Δtrk2Δ mutant was treated with the channel-inactivated derivative C35deOAmB. In contrast, ⁸⁶Rb⁺ uptake was restored when trk1Δtrk2Δ cells were treated with AmB.

### Example 5: Vigor and Sustainability of AmB Ion Channel-Mediated Restoration of Yeast Cell Growth

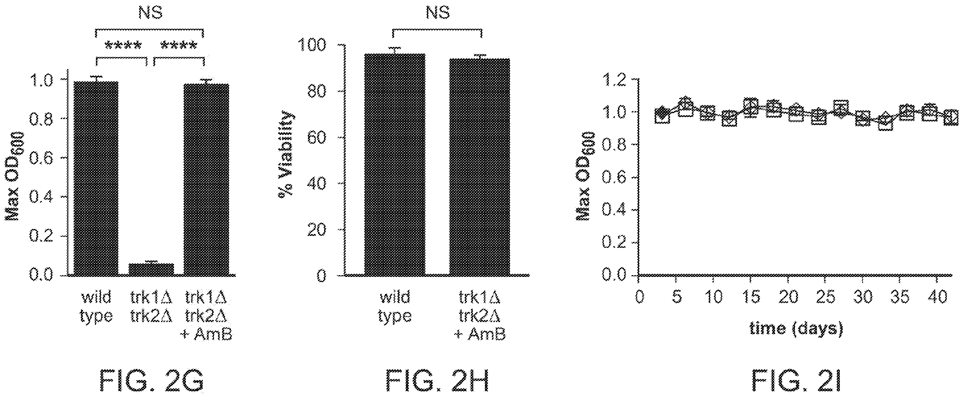

We also quantified the vigor and sustainability of this AmB ion channel-mediated restoration of yeast cell growth. AmB-treated trk1Δtrk2Δ cells reached a maximum cell density that matched that of the wild type (Fig. 2G), and the doubling time for AmB-rescued trk1Δtrk2Δ cells was only 1.7 times longer. Equivalent levels of cell viability in wild type and AmB-rescued trk1Δtrk2Δ cells were also observed (Fig. 2H). To probe the sustainability of this rescue effect, we reiterated the maximum cell density and doubling time experiments for over a month. Cells were passed back and forth every 3 days between solid and liquid media, both containing 125 nM AmB, and the max OD₆₀₀ of liquid cultures 24 h after each liquid inocculation were determined. Like wild type cells, the AmB-rescued trk1Δtrk2Δ cells showed sustained, vigorous cell growth throughout this entire period of time (Fig. 2I). Removing AmB from the media at any point resulted in rapid loss of growth for the trk1Δtrk2Δ cells.

### Example 6: Other Polyene Macrolide Antibiotic Molecules Also Restore Cell Growth in Potassium Channel-Deficient Yeast

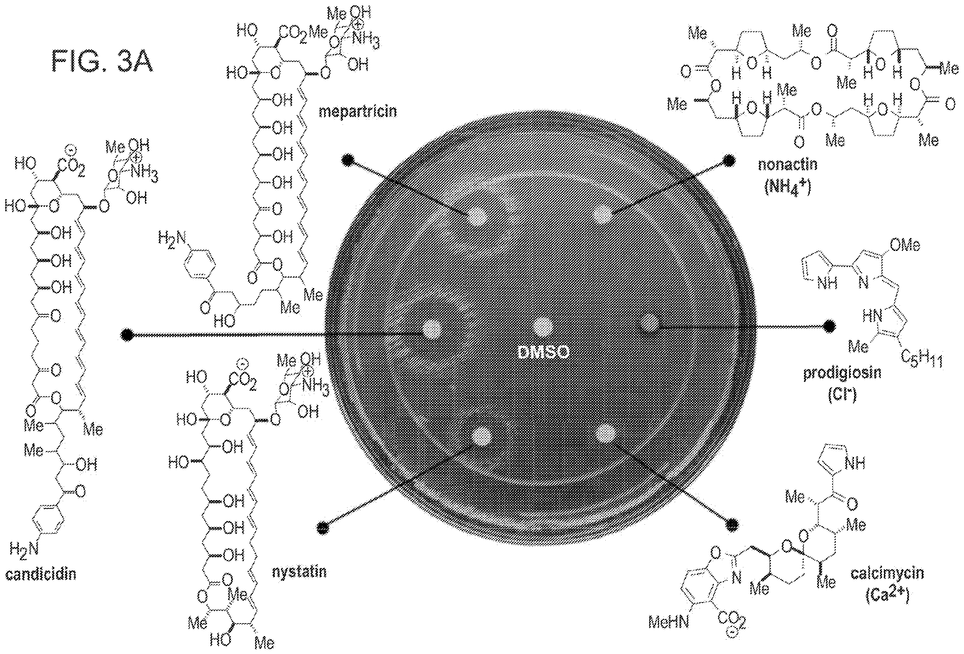

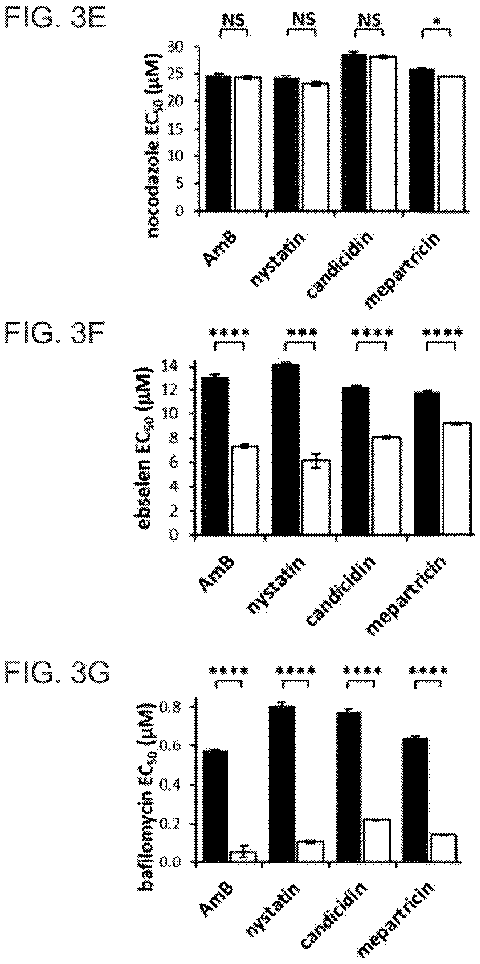

To probe the scope and limitations of this tolerance for imperfect mimicry, a series of additional ion transporting natural products were evaluated. Vigorous restoration of trk1Δtrk2Δ cell growth was observed with other polyene macrolide antibiotic molecules that transport potassium, including nystatin, candicidin, and mepartricin, but not with small molecules that selectively transport NH₄⁺ (nonactin), Cl⁻ (prodigiosin; 4-methoxy-5-[(*Z*)-(5-methyl-4-pentyl-2*H*-pyrrol-2-ylidene)methyl]-1*H*,1'*H*-2,2'-bipyrrole), and Ca²⁺ (calcimycin; A23187; 5-(methylamino)-2-({(2*R*,3*R*,6*S*,8*S*,9*R*,11*R*)-3,9,11-trimethyl-8-[(1*S*)-1-methyl-2-oxo-2-(1*H*-pyrrol-2-yl)ethyl]-1,7-dioxaspiro[5.5]undec-2-yl}methyl)-1,3-benzoxazole-4-carboxylic acid) (Fig. 3A).

### Example 7: Collaboration of Proton Pumps in AmB-Mediated Rescue of Potassium Channel-Deficient Yeast

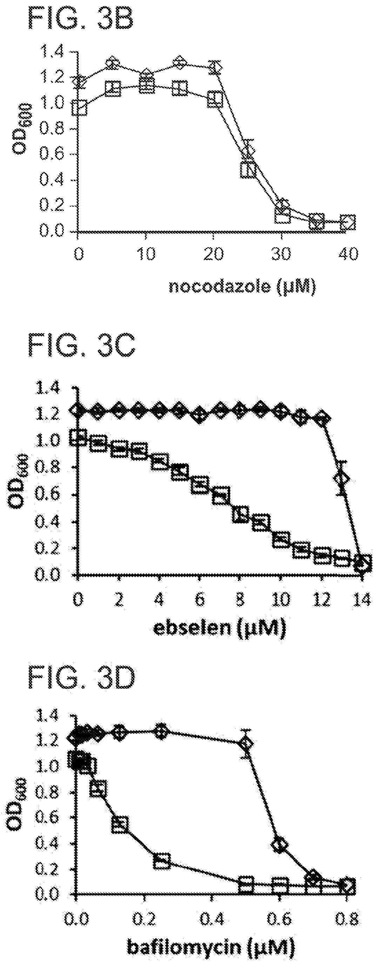

We next probed the mechanistic hypothesis that these potassium channel-forming small molecules restore physiology by collaborating with the V-ATPase and Pma1 proton pumps. Such a model predicts selective sensitivity of the small molecule-rescued mutants to chemical inhibition of these pumps. As a negative control, AmB-treated wild type and AmB-rescued trk1Δtrk2Δ cells were equally sensitive to nocodazole, an off-pathway inhibitor of microtubule dynamics (Fig. 3B). In contrast, AmB-rescued trk1Δtrk2Δ cells were exceptionally sensitive to inhibition of Pma1 with ebselen (Fig. 3C) and V-ATPase with bafilomycin (Fig. 3D). Similar results were observed with nystatin-, candicidin-, and mepartricin-rescued trk1Δtrk2Δ cells (Figs. 3E-G).

### Example 8: AmB Restores Normal Apical Surface Liquid (ASL) Volume in CFTR-Deficient Human Lung Epithelia

We also tested the impact of AmB on apical surface liquid (ASL) volume via confocal fluorescence microscopy. Worthington, E.N. et al. Methods Mol. Biol. 742, 77-92 (2011); Myerburg, M.M. et al. Am. J. Respir. Cell. Mol. Biol. 42, 676-684 (2010). CF lung epithelia were markedly dehydrated relative to normal epithelia. Upon treatment of CF epithelia with AmB (500 nM), normal ASL volume was restored.

### Example 9: AmB Restores Normal Apical Surface Liquid (ASL) Height in CFTR-Deficient Human Lung Epithelia

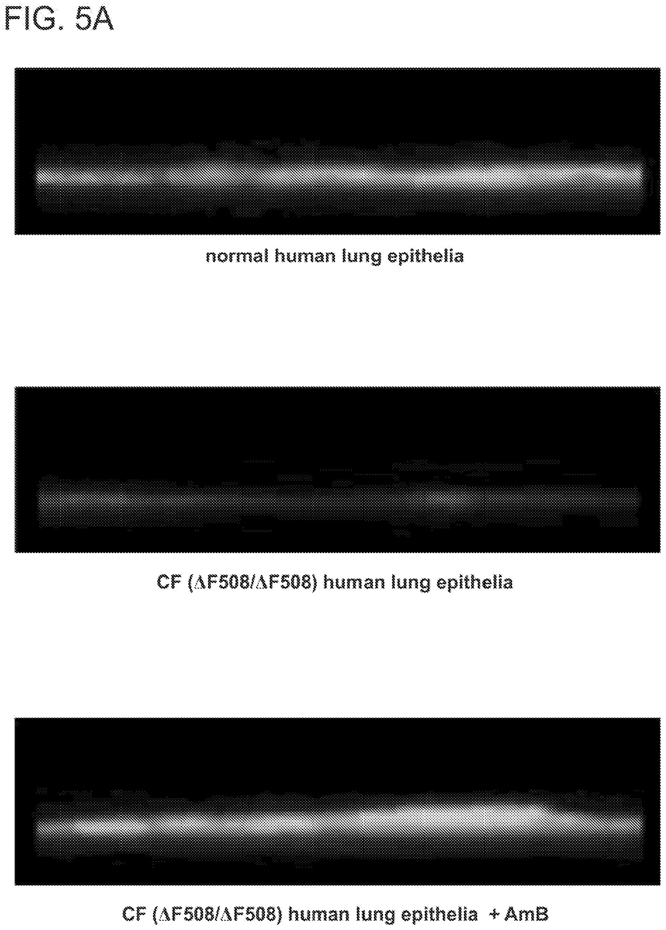

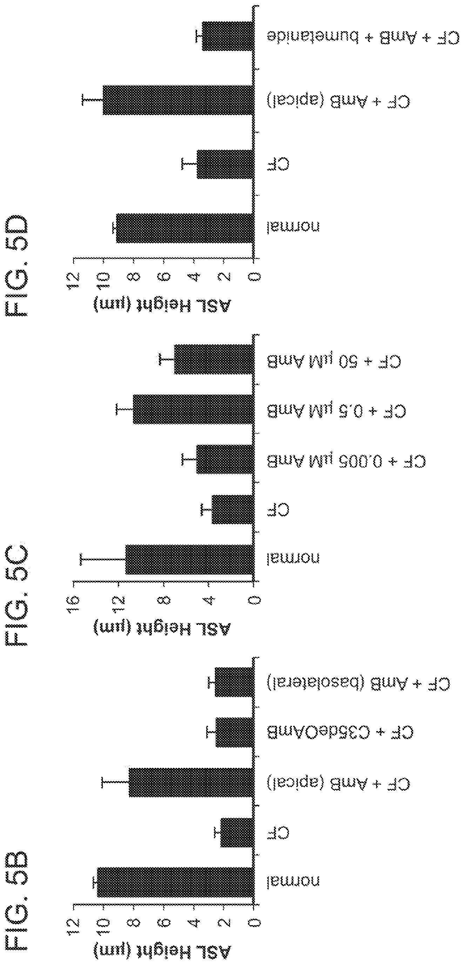

We tested if AmB-mediated permeabilization could also restore ASL height, an important marker for physiology in cystic fibrosis cell line epithelial monolayers, via confocal fluorescence microscopy. NuLi epithelial monolayers (cell line derived from normal human lung epithelia) were treated with vehicle, and CuFi-1 epithelial monolayers (cell line derived from a patient with the most common ΔF508/ΔF508 mutation) were treated with vehicle or AmB. CuFi-1 epithelia were markedly dehydrated relative to NuLi epithelia, consistent with prior reports for ASL height for these cell lines. Upon treatment of CuFi-1 epithelia with AmB (500 nM), normal ASL height was restored (Fig. 5A). Demonstrating that this restoration is specifically caused by AmB permeabilizing the apical membrane, no increase in ASL height was observed upon treatment with the channel-inactivated derivative C35deOAmB, nor by adding AmB to the basolateral surface. These results were quantitatively confirmed using automated ImageJ analysis (Fig. 5B). ASL restoration by AmB was observed in a dose-dependent fashion, with the optimal dose at 0.5 µM AmB (Fig. 5C). This restoration effect was blocked with the basolateral addition of bumetanide to inhibit NKCC, which has been shown previously to reduce ASL in normal lung epithelia (Fig. 5D).

## Claims

1. A pore-forming polyene macrolide for use in treating a disease or condition **characterized by** decreased expression or reduced function of an ion channel, wherein the disease or condition **characterized by** decreased expression or reduced function of an ion channel is selected from the group consisting of cystic fibrosis, hyperkalemic periodic paralysis, paramyotonia congenita, potassium aggravated myotonia, generalized epilepsy with febrile seizures plus (GEFS+), episodic ataxia, familial hemiplegic migraine, spinocerebellar ataxia type 13, long QT syndrome, Brugada syndrome, and mucolipidosis type IV.

2. The pore-forming polyene macrolide for use according to claim 1, wherein the disease or condition **characterized by** decreased expression or reduced function of an ion channel is cystic fibrosis.

3. The pore-forming polyene macrolide for use according to claim 1 or 2, wherein the polyene macrolide is selected from the group consisting of amphotericin B (AmB), nystatin, natamycin, candicidin, and mepartricin, and any combination thereof.

4. The pore-forming polyene macrolide for use according to claim 1 or 2, wherein the polyene macrolide is amphotericin B (AmB).

5. The pore-forming polyene macrolide for use according to any one of the preceding claims, formulated for systemic administration.

6. The pore-forming polyene macrolide for use according to any one of the preceding claims, formulated for pulmonary administration.

7. The pore-forming polyene macrolide for use according to any one of the preceding claims, formulated for administration as an aerosol to an airway of the subject.

## Patentansprüche

1. Porenbildendes Polyenmakrolid zur Verwendung beim Behandeln einer Krankheit oder eines Leidens, die bzw. das durch eine verringerte Expression oder verminderte Funktion eines Ionenkanals gekennzeichnet ist, wobei die Krankheit oder das Leiden, die bzw. das durch eine verringerte Expression oder verminderte Funktion eines Ionenkanals gekennzeichnet ist, aus der Gruppe bestehend aus Mukoviszidose, hyperkaliämischer periodischer Paralyse, Paramyotonia congenita, kaliumsensitiver Myotonie, generalisierter Epilepsie mit Fieberkrämpfen plus (GEFS+), episodischer Ataxie, familiärer hemiplegischer Migräne, spinozerebellärer Ataxie Typ 13, Long-QT-Syndrom, Brugada-Syndrom und Mukolipidose Typ IV ausgewählt ist.

2. Porenbildendes Polyenmakrolid zur Verwendung nach Anspruch 1, wobei die Krankheit oder das Leiden, die bzw. das durch eine verringerte Expression oder verminderte Funktion eines Ionenkanals gekennzeichnet ist, Mukoviszidose ist.

3. Porenbildendes Polyenmakrolid zur Verwendung nach Anspruch 1 oder 2, wobei das Polyenmakrolid aus der Gruppe bestehend aus Amphotericin B (AmB), Nystatin, Natamycin, Candicidin und Mepartricin und einer beliebigen Kombination davon ausgewählt ist.

4. Porenbildendes Polyenmakrolid zur Verwendung nach Anspruch 1 oder 2, wobei das Polyenmakrolid Amphotericin B (AmB) ist.

5. Porenbildendes Polyenmakrolid zur Verwendung nach einem der vorhergehenden Ansprüche, das zur systemischen Verabreichung formuliert ist.

6. Porenbildendes Polyenmakrolid zur Verwendung nach einem der vorhergehenden Ansprüche, das zur pulmonalen Verabreichung formuliert ist.

7. Porenbildendes Polyenmakrolid zur Verwendung nach einem der vorhergehenden Ansprüche, das zur Verabreichung als ein Aerosol an einen Atemweg des Probanden formuliert ist.

## Revendications

1. Macrolide polyénique formant des pores destiné à être utilisé dans le traitement d'une maladie ou d'un trouble **caractérisé par** une expression diminuée ou une fonction réduite d'un canal ionique, dans lequel la maladie ou le trouble **caractérisé par** une expression diminuée ou une fonction réduite d'un canal ionique est choisi dans le groupe constitué par la mucoviscidose, la paralysie périodique hyperkaliémique, la paramyotonie congénitale, la myotonie aggravée par le potassium, l'épilepsie généralisée avec des convulsions fébriles plus (GEFS+), l'ataxie épisodique, la migraine hémiplégique familiale, l'ataxie spinocérébelleuse de type 13, le syndrome du QT long, le syndrome de Brugada, et la mucolipidose de type IV.

2. Macrolide polyénique formant des pores destiné à être utilisé selon la revendication 1, dans lequel la maladie ou le trouble **caractérisé par** une expression diminuée ou une fonction réduite d'un canal ionique est la mucoviscidose.

3. Macrolide polyénique formant des pores destiné à être utilisé selon la revendication 1 ou la revendication 2, dans lequel le macrolide polyénique est choisi dans le groupe constitué par l'amphotéricine B (AmB), la nystatine, la natamycine, la candicidine, et la mepartricin, et toute combinaison de celle-ci.

4. Macrolide polyénique formant des pores destiné à être utilisé selon la revendication 1 ou la revendication 2, dans lequel le macrolide polyénique est l'amphotéricine B (AmB).

5. Macrolide polyénique formant des pores destiné à être utilisé selon l'une quelconque des revendications précédentes, formulé pour une administration systémique.

6. Macrolide polyénique formant des pores destiné à être utilisé selon l'une quelconque des revendications précédentes, formulé pour une administration pulmonaire.

7. Macrolide polyénique formant des pores destiné à être utilisé selon l'une quelconque des revendications précédentes, formulé pour une administration en tant qu'aérosol aux voies respiratoires du sujet.
